# EUROPEAN PATENT APPLICATION

(11) **EP 2 214 002 A1**
(43) Date of publication of application: **04.08.2010**
(21) Application number: 08852541.5
(22) Date of filing: 19.11.2008
(51) Int. Cl.: G01N 21/78, G01N 27/26, G01N 27/416

(54) **BLOOD GLUCOSE LEVEL MEASURING SYSTEM AND MEASUREMENT DATA MANAGING DEVICE**

(30) Priority: 19.11.2007 JP 2007299666; 28.03.2008 JP 2008087204
(71) Applicant: Terumo Kabushiki Kaisha, Shibuya-ku Tokyo 151-0072 (JP)
(72) Inventor: ASAMA, Koichiro, Ashigarakami-gun Kanagawa 259-0151 (JP); SUGAWARA, Yoshihisa, Ashigarakami-gun Kanagawa 259-0151 (JP); MYOUJOU, Hiroyuki, Ashigarakami-gun Kanagawa 259-0151 (JP); HORIGUCHI, Hiroko, Ashigarakami-gun Kanagawa 259-0151 (JP)
(74) Representative: TBK-Patent
(86) International application number: PCT/JP2008/071008
(87) International publication number: WO 2009/066686

(57) **Abstract**

The present invention is made to provide a safe blood glucose measuring system capable of preventing medical accidents such as forgetting to measure the blood glucose and/or forgetting to administer insulin.

An incomplete patient history processing section periodically searches an insulin administration instruction table for blood-glucose-unmeasured patient, and writes blood-glucose-unmeasured patient in a patient history table. A complete patient history processing section writes data from a blood glucose measurement table in the patient history table as blood-glucose-measured patient. An incomplete patient warning processing section periodically searches the patient history table, and provides control to display a warning when finding any blood-glucose-unmeasured patient.

## Description

### Technical Field

The present invention relates to a technique preferably applied to a blood glucose measuring system and a measurement data managing device.
More particularly, the present invention relates to a blood glucose measuring system capable of measuring blood glucose for a plurality of patients and administering insulin to the patients in hospital in a safe and reliable manner.

### Background Art

As is well known, diabetes results from inappropriate secretion of insulin by the pancreas. Thus, it is necessary to measure the blood glucose level for a diabetic patient before meals, and administer insulin to the patient according to the measured level.

Conventionally, there has been a small sized blood glucose measuring device developed, manufactured and marketed by the applicant of the present invention, the blood glucose level being designed for measuring blood glucose of a patient at home by the patient himself/herself or by a family member of the patient. Patent applications relating to the blood glucose measuring device are disclosed in Patent Documents 1 and 2.

[Patent Document 1] Japanese Unexamined Patent Application Publication No.H10-19888
[Patent Document 2] Japanese Unexamined Patent Application Publication No.H10-318928

### Disclosure of the Invention

### Problems to be Solved by the Invention

It has been reported that the blood glucose measuring device designed for home use is actually used as it is in clinical practice in hospitals where many patients are hospitalized.
However, the blood glucose measuring device designed for home use only has a function of measuring blood glucose, and there are no safety measures for preventing accidents such as patient misidentification, duplicated administration of insulin and the like.
Further, in the blood glucose measuring device designed for home use, functions for improving efficiency of medical practice, such as collectively performing blood glucose measurement process and collectively performing insulin administration process on many patients, are not taken into consideration.
Nowadays, a new blood glucose measuring device having improved functions in terms of safety, efficiency and the like is desired in order to meet the needs of medical practice.

Since many patients must be handled in medical practice, the blood glucose measuring device for medical use needs to include not only a blood glucose measuring device by itself, but also a device for managing blood glucose measurement data and insulin administration data collected by the blood glucose measuring device for each of the patients.
Further, in order to prevent medical accidents such as forgetting to measure blood glucose and/or forgetting to administer insulin, not only the blood glucose measuring device single body needs to be provided with a function for preventing the medical accidents, but also the device for managing data needs to be provided with a warning function.

The present invention is made in view of the above-mentioned problems, and an object of the present invention is to provide a safe blood glucose measuring system capable of preventing medical accidents such as forgetting to measure blood glucose and/or forgetting to administer insulin.

When the blood glucose measuring device is used in hospitals, in many cases a nurse performs the blood glucose measurement for a plurality of patients. Further, in many cases insulin administration is also performed at the same time.
As is well known, work burden of nurses is very heavy in hospitals, and therefore accidents such as forgetting to measure blood glucose for the plurality of patients and/or forgetting to administer insulin to the plurality of patients are prone to occur.

To solve these problems, another object of the present invention is to provide a new measurement data managing device and blood glucose measuring system capable of rapidly giving a warning that blood glucose measurement for the plurality of patients and/or insulin administration to the plurality of patients is forgotten, and rapidly performing these processes again.

To solve the aforesaid problems, a blood glucose measuring system according to an aspect of the present invention comprises: a blood glucose meter; and a measurement data managing device, wherein the blood glucose meter includes: a measuring portion for measuring blood glucose level; a nonvolatile storage adapted to store a blood glucose measurement table for recording the measured blood glucose level; and a transmitting section adapted to transmit the blood glucose measurement table, and wherein the measurement data managing device includes: a insulin administration instruction table having a patient ID field, and a scheduled measurement time field for performing blood glucose measurement; a patient history table having a patient ID field, a scheduled measurement date and time field, a measurement date and time field, a blood glucose level field, and a incomplete flag field; a receiving section for being connected with the blood glucose meter to receive the blood glucose measurement table from the blood glucose meter; a complete patient history processing section adapted to record a record acquired from the blood glucose measurement table in the patient history table as a record whose incomplete flag field is "false"; an incomplete patient history processing section adapted to additionally record a record, which is obtained by searching the scheduled measurement time field of the insulin administration instruction table for the patient ID scheduled to receive a blood glucose measurement in a period between the current time and a predetermined time ago, in the patient history table as a record whose incomplete flag field is "true"; an incomplete patient warning processing section adapted to search the patient history table to judge whether or not there is any record whose incomplete flag field is "true"; a display control section adapted to create a warning in the case where the incomplete patient warning processing section judges that there is a record whose incomplete flag field is "true" in the patient history table; and a display unit adapted to display the warning created by the display control section.

Further, to solve the aforesaid problems, a measurement data managing device according to another aspect of the present invention comprises: a patient information table having a round type in process field in which an instruction on whether or not blood glucose measurement shall be performed and whether or not drug administration shall be performed is recorded for each patient, the instruction being contained in patient data transmitted to a blood glucose meter; a patient history table having a drug administration result field in which results of performing the blood glucose measurement and the drug administration are recorded for each patient, the results being contained in patient's measurement data received from the blood glucose meter; and a control section adapted to compare the round type in process field with the drug administration result field, and, if there is any discrepancy, create necessary data for performing the blood glucose measurement or the drug administration for each patient associated with the discrepancy, as well as create a warning message, and transmit the created data to the blood glucose meter.

The incomplete patient history processing section periodically searches the insulin administration instruction table for the blood-glucose-unmeasured patient, and writes the blood-glucose-unmeasured patient in the patient history table. At this time, the incomplete flag field of the record additionally recorded in the patient history table is set to "true".
In contrast, the complete patient history processing section writes the record of the blood-glucose-measured patient from the blood glucose measurement table downloaded from the blood glucose meter in the patient history table. At this time, the incomplete flag field of the record additionally recorded in the patient history table is set to "false".
The incomplete patient warning processing section periodically searches the patient history table, and provides control to display a warning when finding any record whose incomplete flag field is "true".
The process of the complete patient history processing section precedes the process of the incomplete patient history processing section. The record additionally recorded previously by the incomplete patient history processing section is overwritten by the complete patient history processing section.

The measurement data managing device holds the type information of the content to be performed, which is contained in the data transmitted from the measurement data managing device to the blood glucose meter, in the patient information table thereof. Further, after reading and writing the data received from the blood glucose meter in the patient history table, the measurement data managing device verifies whether or not blood glucose measurement and/or insulin administration has been performed according to the instruction. As a result of the verification, in there is any discrepancy, it can be judged that blood glucose measurement and/or insulin administration is forgotten to be performed. Based on this judgment, the measurement data managing device creates a warning message, creates the patient whose blood glucose measurement and/or insulin administration is forgotten to be performed and the content due to be performed on the patient again, and transmits the round data to the blood glucose meter.

### Advantages of the Invention

According to the present invention, it is possible to provide a safe blood glucose measuring system capable of preventing medical accidents such as forgetting to measure the blood glucose and/or forgetting to administer insulin.

Further, according to the present invention, it is possible to provide a new measurement data managing device and blood glucose measuring system capable of rapidly giving a warning that blood glucose measurement for the plurality of patients and/or insulin administration to the plurality of patients is forgotten, and rapidly performing these processes again.

### Brief Description of Drawings

FIG. 1 is a schematic view showing the overall configuration of a blood glucose measuring system according to an embodiment of the present invention.
FIGS. 2A and 2B are perspective views showing the appearance of a blood glucose meter according to the aforesaid embodiment of the present invention.
FIGS. 3A, 3B 3C and 3D are views showing the blood glucose meter according to the aforesaid embodiment when viewed from four directions.
FIGS. 4A and 4B are views showing the appearance of a cradle in a state when the blood glucose meter is removed therefrom.
FIGS. 5A and 5B are views showing the appearance of the cradle in a state when the blood glucose meter is mounted thereon.
FIG. 6 is a block diagram showing the internal configuration of the blood glucose meter.
FIG. 7 is a block diagram showing the internal configuration of the cradle.
FIG. 8 is a block diagram showing connection state of the blood glucose meter, the cradle and a measurement data managing device.
FIG. 9 is a block diagram showing the measurement data managing device.
FIG. 10 is a functional block diagram of the measurement data managing device.
FIG. 11 shows an example of tables arranged in the measurement data managing device.
FIGS. 12A and 12B are views showing a top menu window displayed on a display unit of the measurement data managing device.
FIG. 13 is a functional block diagram of a complete patient history processing section.
FIG. 14 shows fields of a blood glucose measurement table received from the blood glucose meter.
FIG. 15 is a flowchart showing the operation flow of the complete patient history processing section.
FIG. 16 is a flowchart showing the operation flow of a patient history table recording process.
FIG. 17 is a schematic view showing a warning window.
FIG. 18 is a functional block diagram of an incomplete patient history processing section.
FIG. 19 is a flowchart showing the operation flow of the incomplete patient history processing section.
FIG. 20 is a functional block diagram of an incomplete patient warning processing section.
FIG. 21 is a flowchart showing the operation flow of the incomplete patient warning processing section.
FIG. 22 is a flowchart showing the operation flow of an incomplete list display process.
FIG. 23 is a view showing the state of the top menu window after a predetermined time has elapsed from the scheduled blood glucose measurement time.
FIG. 24 is a view showing an incomplete patient list window.
FIG. 25 is a view schematically showing the flow of blood glucose measurement operation performed using the blood glucose meter according to an embodiment of the present invention.
FIG. 26 is a view showing internal constitution of each of tables and correlation between the tables.
FIG. 27 is a flowchart showing the flow of the communication between the blood glucose meter and the measurement data managing device.
FIG. 28 is a flowchart showing the continuation of the flow of the communication between the blood glucose meter and the measurement data managing device.
FIG. 29 is a functional block diagram of the blood glucose meter.
FIG. 30 is a state transition diagram of the blood glucose meter.
FIG. 31 is a flowchart showing the processing flow of the blood glucose meter.
FIG. 32 is a flowchart showing the continuation of the processing flow of the blood glucose meter.
FIG. 33 is a functional block diagram of the measurement data managing device.
FIG. 34 is a flowchart showing the processing flow when patient data or the like is transferred from the measurement data managing device to the blood glucose meter.
FIG. 35 is a flowchart showing the processing flow when the measurement data managing device receives patient's measurement data or the like from the blood glucose meter.
FIG. 36 is a flowchart showing the continuation of the processing flow when the measurement data managing device receives patient's measurement data or the like from the blood glucose meter.
FIG. 37 is a view showing a main menu window displayed on the display unit of the measurement data managing device.
FIG. 38 is a view schematically showing change of the flags contained in the data and tables existing between the measurement data managing device and the blood glucose meter.

### Best Modes for Carrying Out the Invention

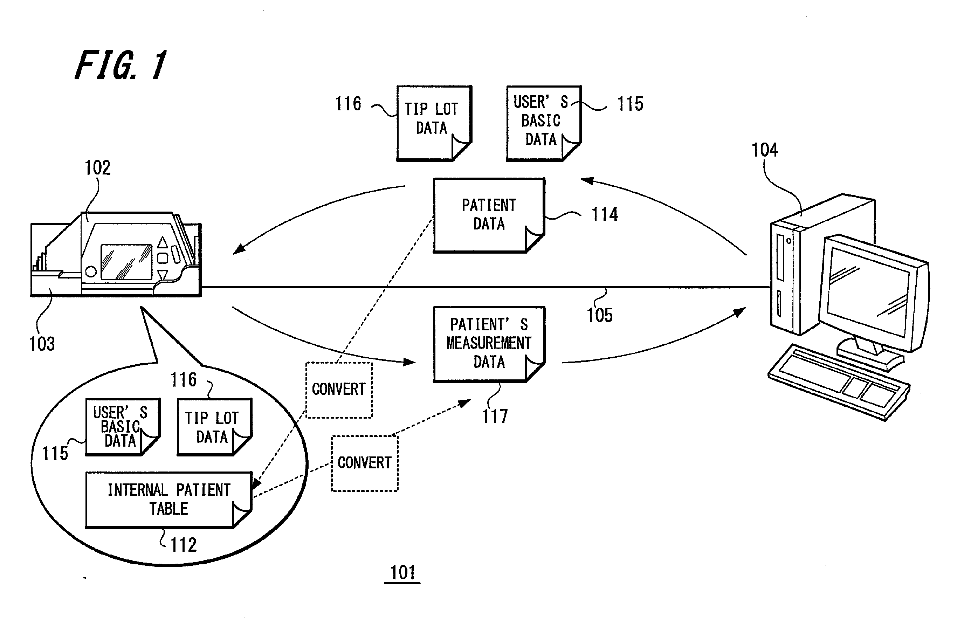

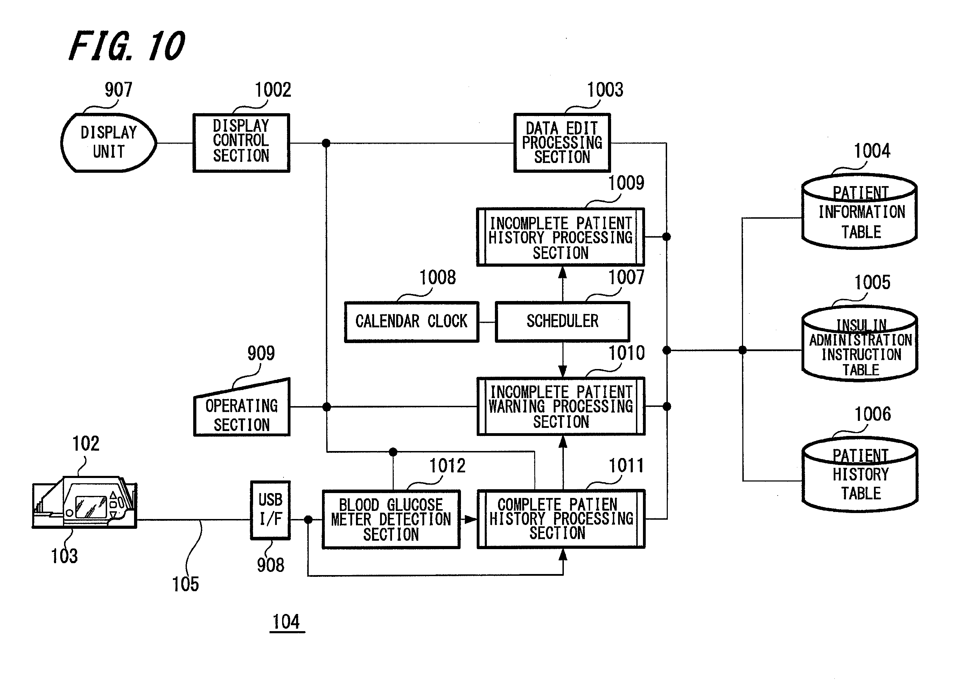

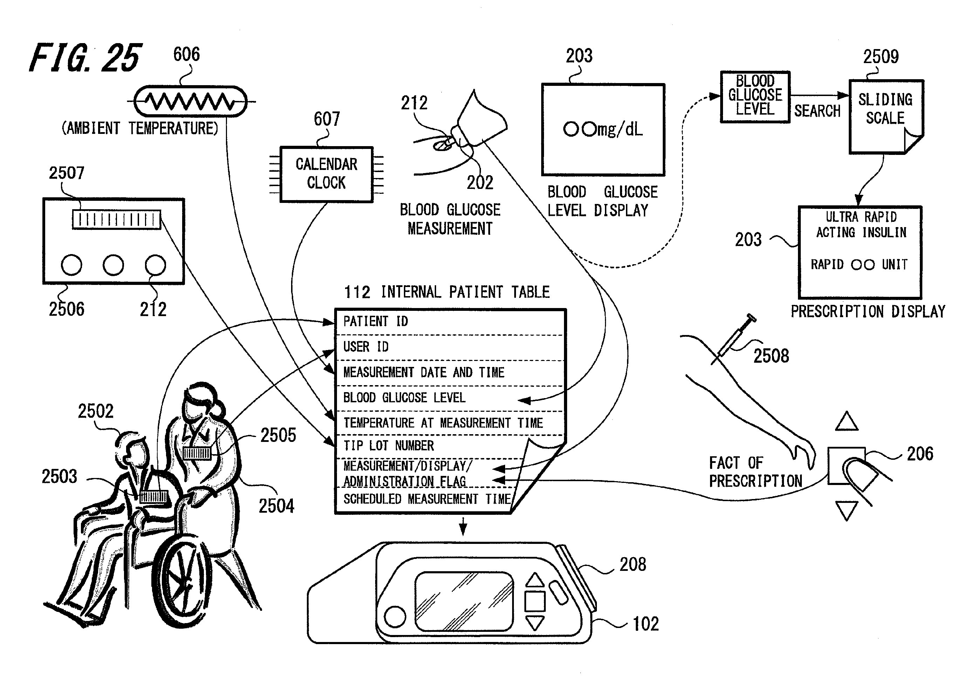

An embodiment of the present invention will be described below with reference to FIGS. 1 to 44.
Incidentally, in the present embodiment, two embodiments are described.
First, common configuration of the two embodiments will be described with reference to FIGS. 1 to 9.
Next, a first embodiment will be described with reference to FIGS. 10 to 24.
Next, a second embodiment will be described with reference to FIGS. 25 to 38.

### [Blood Glucose Measuring System 101]

FIG. 1 is a schematic view showing the overall configuration of a blood glucose measuring system according to an example of the embodiments of the present invention.
A blood glucose measuring system 101 includes a blood glucose meter 102, which is a blood glucose measuring device, a cradle 103 and a measurement data managing device 104.
The blood glucose meter 102 is a portable device that basically fits in the palm of an adult hand and that is operated using a secondary battery such as a lithium-ion secondary battery.
In the case where the blood glucose level of a patient is measured by a doctor, a nurse or the like, generally the blood glucose meter 102 is brought into a ward of a hospital, and a tiny amount of blood is drawn from the patent to measure the blood glucose level.
Make sure that the blood glucose meter 102 is mounted on the cradle 103 after performing the blood glucose measurement and insulin administration.
In addition to charging the battery of the blood glucose meter 102, the cradle 103 also functions as an interface through which the blood glucose meter 102 transmits/receives data to/from the measurement data managing device 104.
The partner to/from which the blood glucose meter 102 transmits/receives data through the cradle 103 is the measurement data managing device 104.

The measurement data managing device 104, which is configured by a personal computer, is connected with the cradle 103 through a USB cable 105.
A known OS operates to execute the measurement data managing device 104. Further, a program for causing the computer to function as the measurement data managing device 104 operates under the OS.

As soon as the blood glucose meter 102 is mounted on the cradle 103, the communication between the blood glucose meter 102 and the measurement data managing device 104 is performed through the cradle 103. At this time, if there is an internal patient table 112 in the blood glucose meter 102, the measurement data stored in the internal patient table 112 will be converted into patient's measurement data 117 and then transmitted to the measurement data managing device 104 immediately.
Further, by transmitting a predetermined command from the measurement data managing device 104, blood glucose meter setting data (not shown in the drawings) can be downloaded from the blood glucose meter 102 to the measurement data managing device 104.
Further, patient data 114, user's basic data 115 and tip lot data 116 can be uploaded to the blood glucose meter 102 from the measurement data managing device 104.
The details about these data will be described later.

### [Appearance: Blood Glucose Meter 102]

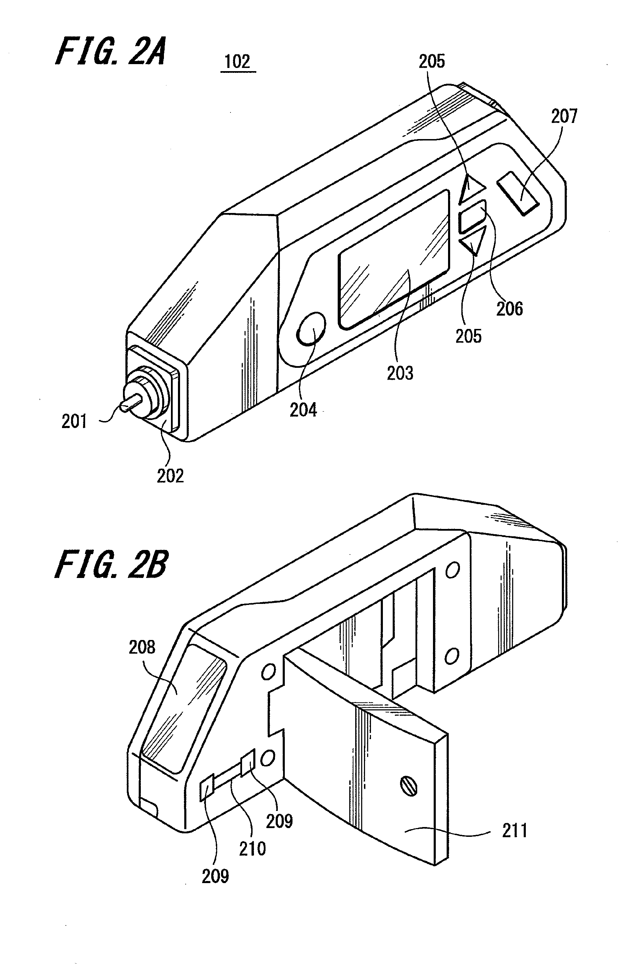

FIGS. 2A and 2B are perspective views showing the appearance of the blood glucose meter 102.

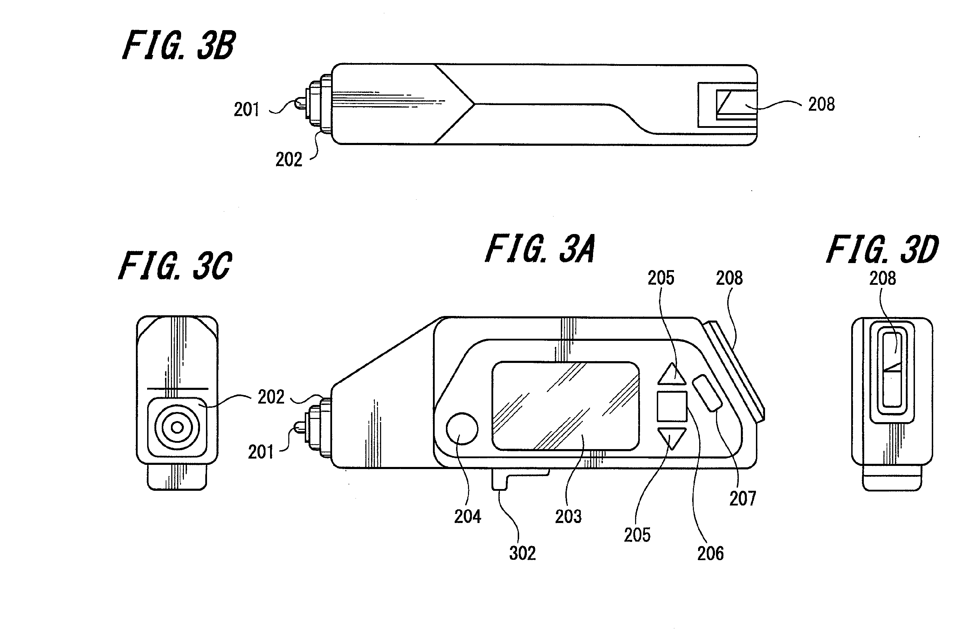

FIGS. 3A, 3B 3C and 3D are views showing the blood glucose meter when viewed from four directions.
In order to facilitate description, the surface where an LCD is provided (as shown in FIGS. 2A and 3A) is referred to as a "body front surface" hereinafter, and the surface where a battery lid is provided (as shown in FIGS. 2B) is referred to as a "body rear surface".

As shown in FIGS. 3A and 3C, an optical measuring section 202 is provided at the tip end of the blood glucose meter 102.
The optical measuring section 202 has a shape allowing a blood glucose measuring tip 212 (referred to as "measuring tip 212" hereinafter) to be attached and detached. The used measuring tip 212 can be detached from the optical measuring section 202 by operating an eject lever 302.
As shown in FIG. 2A and FIG. 3A, a power switch 204, Cursor keys 205, an Enter key 206 and a bar-code key 207 are arranged on the side surface (the body front surface) where the LCD 203 (a liquid crystal display) is provided, at positions beside the LCD 203.
The power switch 204 is used to switch on and off the power of the blood glucose meter 102.
The Cursor keys 205 are used to move the cursor to select one of a plurality of items displayed on the LCD 203.
The Enter key 206 is used to issue an instruction for "executing" or "selecting" the item selected by the cursor.
The bar-code key 207 is used to cause a bar-code reader 208 shown in FIG. 3D to operate, wherein the bar-code reader 208 is arranged on a side of the blood glucose meter 102 opposite to the side where the optical measuring section 202 is arranged.
The bar-code reader 208 is a bar-code reading device configured by a combination of a known red laser diode and a light-receiving element such as a phototransistor. Incidentally, an image sensor such as a CCD, a CMOS or the like can be used instead of the light-receiving element.

The basic mechanism of the blood glucose meter 102 for measuring blood glucose is identical to that of the conventional arts, and will be briefly described below.
The measuring tip 212 is attached to the optical measuring section 202, the fingertip of the patient is stuck with a puncture tool, and the blood exuded from the fingertip is soaked into the measuring tip 212. A test paper made of a porous membrane such as a polyethersulfone membrane is provided inside the measuring tip 212. Further, when the blood soaked into the measuring tip 212 is permeated into the test paper, the blood will be reacted with the reagent contained in the test paper, so that the test paper develops a color. The color reaction requires about several to ten and several seconds, and the reaction time is affected by the ambient temperature.
After a predetermined reaction time has elapsed, a light-emitting element is caused to emit light, the light emitted from the light-emitting element irradiates to the test paper, and the light reflected from the test paper is received by the light-receiving element. Further, an analog light-receiving intensity signal obtained from the light-receiving element is converted into a digital value, and thereafter the digital value is converted into the blood glucose level to be displayed on the LCD 203.
Incidentally, the mechanism of blood glucose measurement on the side of the blood glucose meter 102 is not limited to the aforesaid optical measurement method in which a coloring reagent is used, but may be any other methods possible be used to perform the conventional blood glucose measurement, such as an electrochemical sensor method or the like.

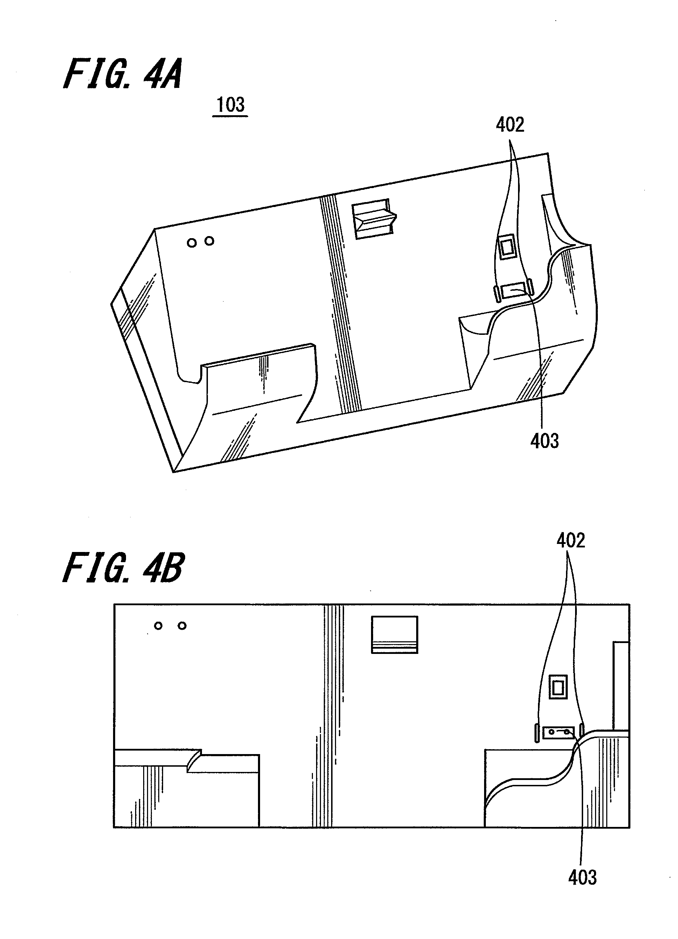

As shown in FIG. 2B, a power terminal 209 and an infrared communication window 210 are provided in the body rear surface on the side of the bar-code reader 208. When the blood glucose meter 102 is mounted on the cradle 103, the power terminal 209 is brought into contact with a charging terminal 402 (see FIGS. 4A and 4B) provided in the cradle 103, so that the blood glucose meter 102 is charged while an infrared communication between the blood glucose meter 102 and the cradle 103 is performed. Incidentally, the battery lid 211 is also provided on the body rear surface.

### [Appearance: Cradle 103]

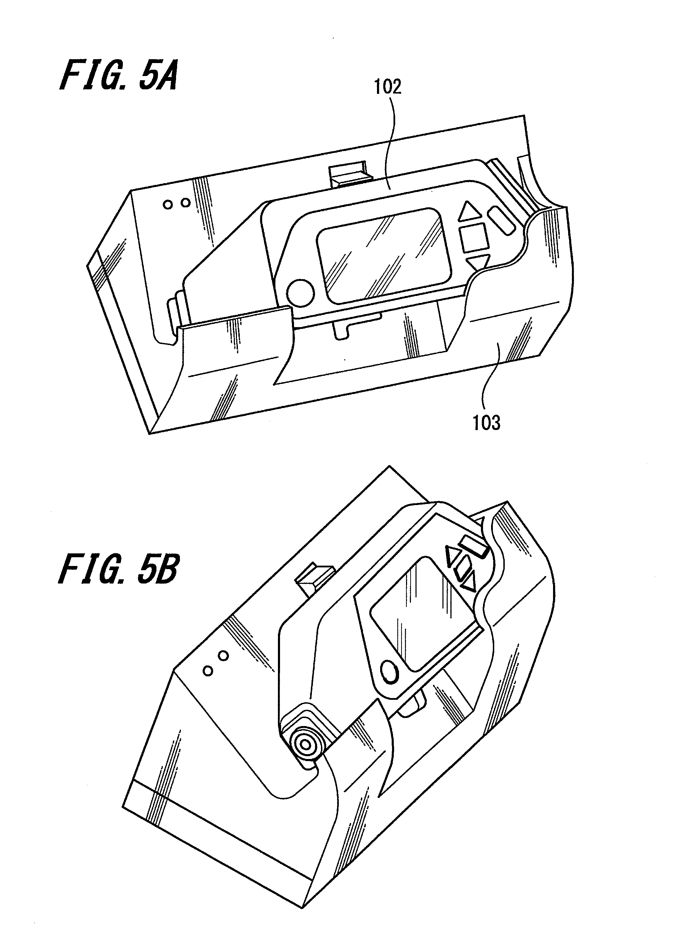

FIGS. 4A and 4B are views showing the appearance of the cradle 103 in a state when the blood glucose meter 102 is removed therefrom, and FIGS. 5A and 5B are views showing the appearance of the cradle 103 in a state when the blood glucose meter 102 is mounted thereon.
As shown in FIGS. 4A and 4B, the charging terminal 402 is arranged in the cradle 103 at a position corresponding to the power terminal 209 of the blood glucose meter 102. Similarly, an infrared communication window 403 is arranged in the cradle 103 at a position corresponding to the infrared communication window 210 of the blood glucose meter 102.
An infrared light-emitting diode and a phototransistor are provided inside both the infrared communication window 210 of the blood glucose meter 102 and the infrared communication window 403 of the cradle 103. These components constitute a known IrDA (Infrared Data Association) based infrared serial communication interface.

As shown in FIG. 1, the cradle 103 is connected to the measurement data managing device 104 through the USB cable 105. The cradle 103 has a function of charging the battery of the blood glucose meter 102. Since it is possible to connect many cradles 103 to the measurement data managing device 104, the cradle 103 is configured as a self-powered device which does not receive power supply from a USB terminal of the measurement data managing device 104.

### [Hardware: Blood Glucose Meter 102]

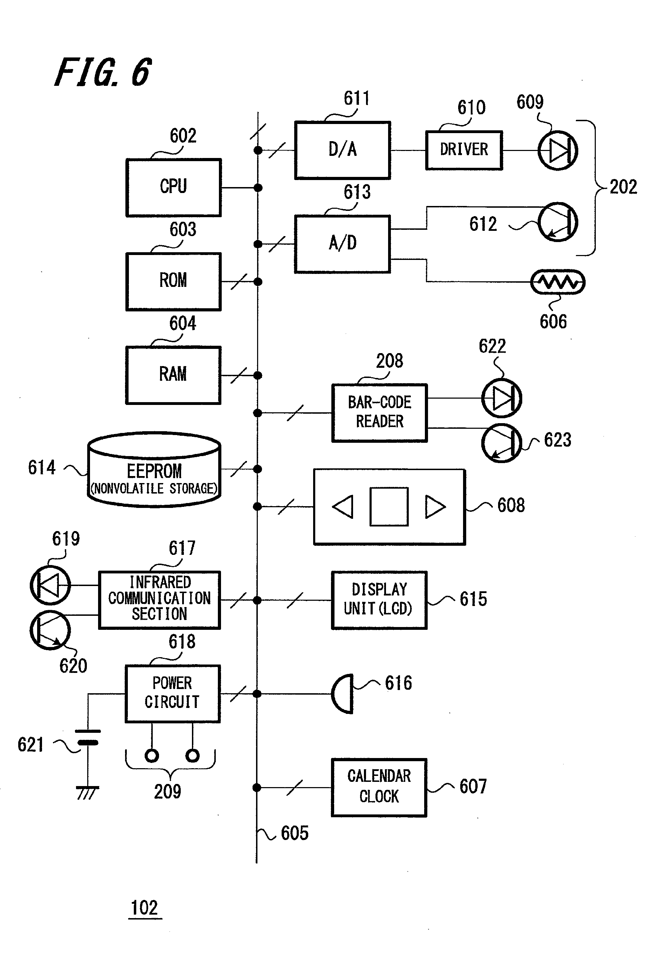

FIG. 6 is a block diagram showing the internal configuration of the blood glucose meter 102.
The blood glucose meter 102 includes a CPU 602, a ROM 603, a RAM 604, and a bus 605 for connecting the CPU 602, the ROM 603 and the RAM 604. In addition to the aforesaid components, a section for providing a data input function and a section for providing a data output function are also connected to the bus 605.
For sake of convenience, hereinafter the CPU 602, the ROM 603, the RAM 604 and the bus 605 are referred to as a microcomputer that constitutes the blood glucose meter 102.

The section for providing a data input function of the blood glucose meter 102 includes the optical measuring section 202 for obtaining blood glucose measurement data, a thermistor 606 for obtaining temperature data, the bar-code reader 208, a calendar clock 607, and an operating section 608.
The optical measuring section 202 includes a light-emitting portion and a light-receiving portion, wherein the light-emitting portion includes a light-emitting diode 609, a driver 610 of the light-emitting diode 609, and a D/A converter 611 connected to the driver 610, and the light-receiving portion includes a phototransistor 612 and an A/D converter 613.
Since the test paper arranged inside the measuring tip 212 needs to be irradiated by light of a suitable intensity, the light-emitting diode 609 is controlled so as to emit light based on light-emitting intensity data stored in a below-mentioned nonvolatile storage 614. In other words, the light-emitting intensity data is read out from the nonvolatile storage 614, converted into an analog voltage signal by the D/A converter 611, and then power-amplified by the driver 610 to drive the light-emitting diode 609 to emit light.
On the other hand, a signal voltage of intensity of the light received by the phototransistor 612 is converted into digital data by the A/D converter 613. Further, the converted digital data is converted into blood glucose level data by performing a predetermined arithmetic process executed by the CPU 602, and then the blood glucose level data is recorded in a predetermined area of the RAM 604 and a predetermined area of the nonvolatile storage 614.

Further, the blood glucose meter 102 has the thermistor 606, and ambient temperature of the blood glucose meter 102 can be measured based on the change of the resistance of the thermistor 606. Similar to the phototransistor 612, the resistance of the thermistor 606 is converted into digital data by the A/D converter 613, and the digital data is stored in a predetermined area of the RAM 604 and a predetermined area of the nonvolatile storage 614. Incidentally, since it is not necessary to simultaneously measure the light-receiving intensity and the temperature, the A/D converter 613 can be shared by the phototransistor 612 and the thermistor 606.

The bar-code reader 208 causes a red laser diode 622 to emit light, the reflected light is received by a phototransistor 623 so that the bar-code is read, and the data recorded on the bar-code is outputted to the bus 605.

The calendar clock 607 is a known IC also called as a "real-time clock" which provides a date and time data output function, and is mounted as standard on many microcomputers, personal computers and the like.
In the blood glucose meter 102 according to the present embodiment of the present invention, since it is necessary to acquire information regarding the date and time when the blood glucose was being measured, date and time information is an important information. In other words, the data to be collected and the date and time information have very close relation with each other. Further, the date and time information when the blood glucose was being measured needs to be stored in the internal patient table 112 along with the blood glucose level. For this reason, the calendar clock 607 is daringly shown in the drawings.

The operating section 608 is a known key switch formed by push-buttons, the operating section 608 including the Cursor keys 205 and the Enter key 206. The operating section 608 is used for a user to operate the blood glucose meter 102 according to the content displayed on a below-mentioned display unit 615 which is a LCD.

The section for providing a data output function of the blood glucose meter 102 includes the display unit 615 configured by the LCD 203, a buzzer 616 and an infrared communication section 617.
Various screens are displayed on the display unit 615 by a program stored in the ROM 603 and executed by the CPU 602. The details about these screens will be described later.
The buzzer 616 is mainly used to notify the user that the bar-code reader 208 has successfully read the bar-code, that the measurement operation of the blood glucose measurement has completed, that the infrared communication has completed, or that an error message is displayed. The buzzer 616 may also sound every time when operating the operating section 608 depending on setting.
As mentioned above, an infrared light-emitting diode 619 and a phototransistor 620 are connected to the infrared communication section 617, and these components constitute an IrDA based serial interface. When detecting that the blood glucose meter 102 has received power supply from the cradle 103 based on voltage changing of the power terminal 209, a power circuit 618 reports this fact to the CPU 602 through the bus 605. Further, based on the control of the CPU 602, the infrared communication function of the infrared communication section 617 is started, so that the infrared communication between the blood sugar meter 102 and the cradle 103 is performed, and thereby the various tables stored in the nonvolatile storage 614 transmits/receives data to/from the measurement data managing device 104 and is updated.
In other words, when performing the infrared communication between the blood glucose meter 102 and the cradle 103, the infrared communication is immediately executed as soon as the blood glucose meter 102 is mounted on the cradle 103 without needing to operate the operating section 608 and the like of the blood glucose meter 102.

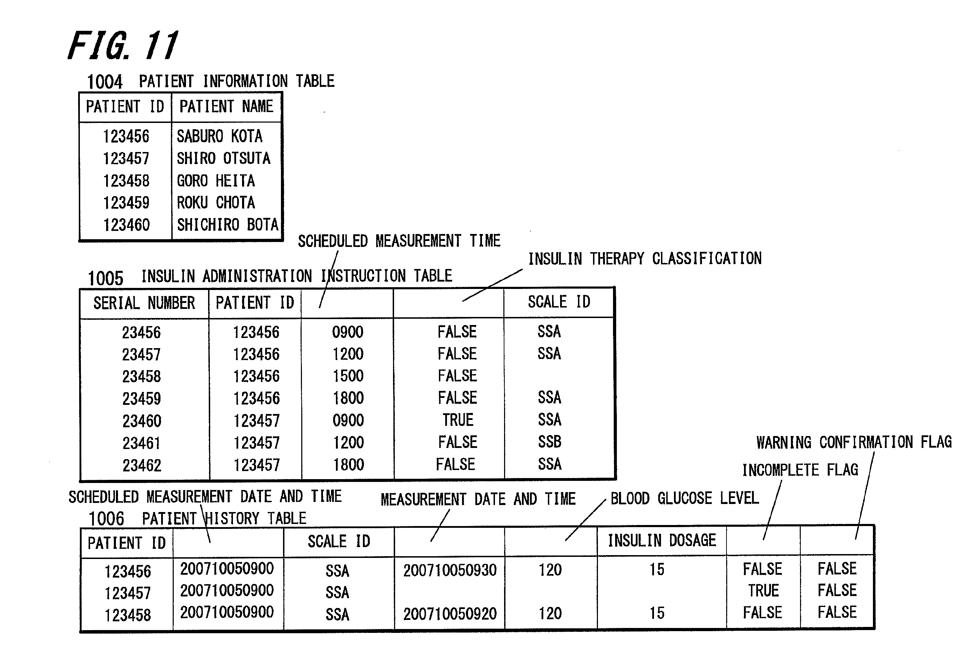

In addition to the data output function, the blood glucose meter 102 is provided with the nonvolatile storage 614 (which is an EEPROM) that provides data storage function. A patient table 1109, a user table 1113, a tip lot table 1117, a prescription information table 1502, a measurement/prescription table 1602, a measurement/prescription results table 1408 and the like (all these tables are to be described later with reference to FIGS. 11, 14, 15 and 16) are stored in the nonvolatile storage 614. These tables are updated when the communication between the blood glucose meter 102 and the measurement data managing device 104 is performed through the cradle 103. Incidentally, a flash memory may also be used instead of the EEPROM.

### [Hardware: Cradle 103]

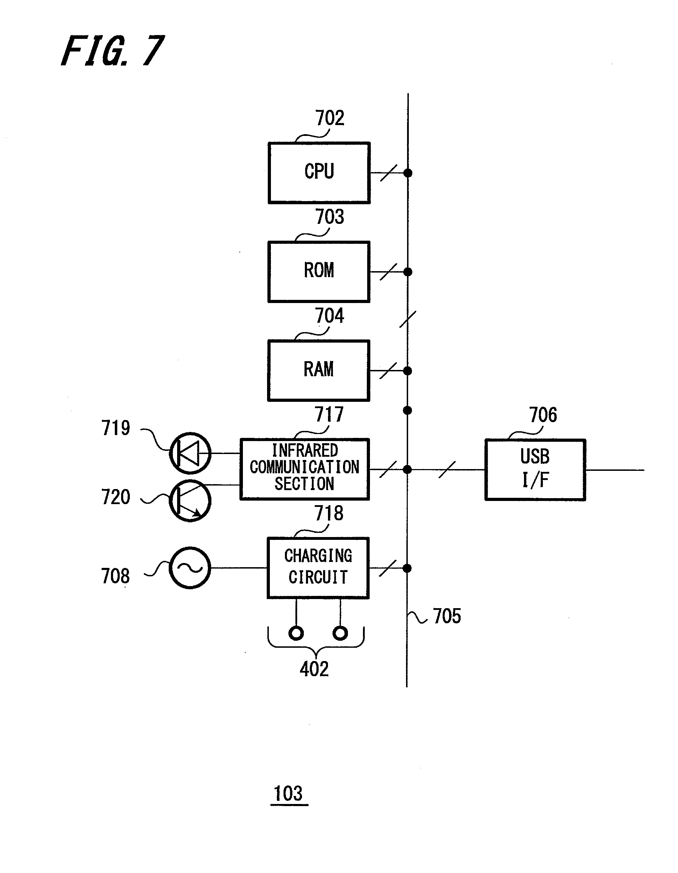

FIG. 7 is a block diagram showing the internal configuration of the cradle 103.

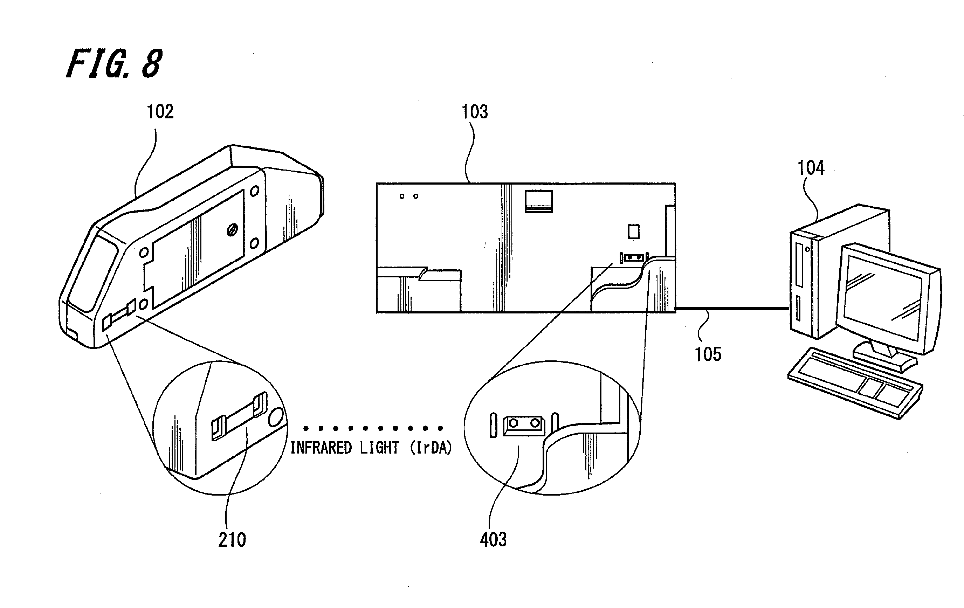

FIG. 8 is a view schematically showing connection state of the blood glucose meter 102, the cradle 103 and the measurement data managing device 104.
As shown in FIG. 7, the cradle 103 includes a CPU 702, a ROM 703, a RAM 704, an infrared communication section 717, a USB interface (I/F) 706, a charging circuit 718 and a bus 705 which connects these components, wherein the CPU 702, the ROM 703 and the RAM 704 constitute a microcomputer, and the infrared communication section 717 has an infrared light-emitting diode 719 and a phototransistor 720 connected thereto.
When detecting that the blood glucose meter 102, which is a load, is connected based on voltage changing of the charging terminal 402, the charging circuit 718 reports this fact to the CPU 702 through the bus 705. Further, based on the control of the CPU 702, the infrared communication function of the infrared communication section 717 is started, so that the communication between the blood glucose meter 102 and the measurement data managing device 104 is performed through the infrared communication section 717 and the USB interface 706.

As described above, the blood glucose meter 102 and the cradle 103 are connected with each other through the IrDA, and the cradle 103 and the measurement data managing device 104 are connected with each other through the USB. From this aspect, the cradle 103 serves as an interface to enable data communication between the measurement data managing device 104 and the blood glucose meter 102.

### [Hardware: Measurement Data Managing Device 104]

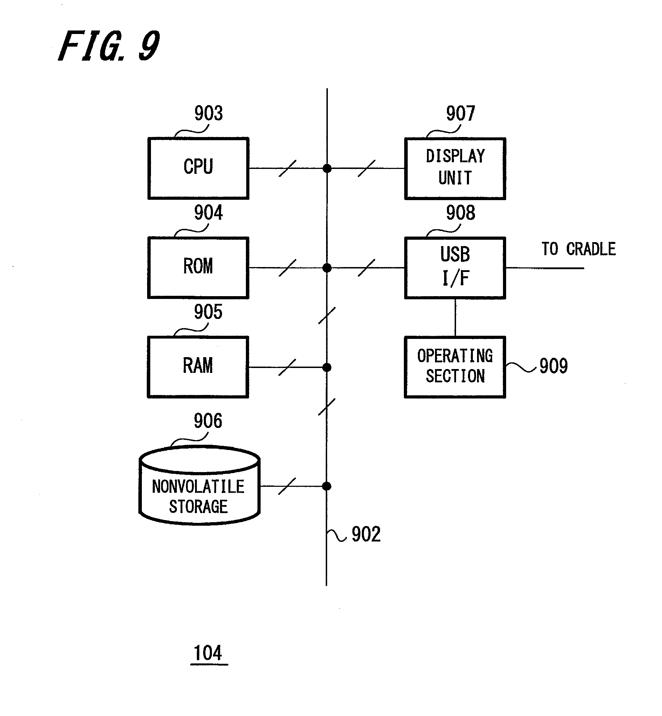

FIG. 9 is a block diagram showing the measurement data managing device 104.
As described above, the measurement data managing device 104 is actually a known personal computer.
A bus 902 is provided inside the measurement data managing device 104. A CPU 903, a ROM 904, a RAM 905, a nonvolatile storage (such as a hard disk device or the like) 906, a display unit (such as a LCD or the like) 907 and a USB interface (I/F) 908 are connected to the bus 902. In addition to an operating section (such as a keyboard, a mouse and/or the like) 909, the cradle 103 is connected to the USB interface 908.

### [First Embodiment]

### [Software: Measurement Data Managing Device 104]

FIG. 10 is a functional block diagram of the measurement data managing device 104. FIG. 10 schematically shows the function of the program for realizing the measurement data management function.
A window of an operation panel or the like (as a user interface) created by a display control section 1002 is displayed on the display unit 907, which is a display.
Operating information generated from the operating section 909 (such as a keyboard, a mouse and/or the like) is transmitted to each of function sections.
A data edit processing section 1003 operates data of a patient information table 1004, an insulin administration instruction table 1005, a patient history table 1006 and the like.
A scheduler 1007 acquires the date and time information from a calendar clock 1008 in real time, and executes designated programs according to a schedule table provided therein. The designated programs mean an incomplete patient history processing section 1009 and an incomplete patient warning processing section 1010 (Note: the term "incomplete" used herein and hereinafter means blood glucose is unmeasured and/or insulin is unadministered).

The incomplete patient history processing section 1009 is activated by the scheduler 1007 at a predetermined time interval. In the present embodiment, the predetermined time interval m can be changed by the data edit processing section 1003. For example, the predetermined time interval m can be set to 60 minutes.
The incomplete patient history processing section 1009 performs the following processes.
· Collecting/creating scheduled blood glucose measurement date and time data of the patient whose blood glucose is judged not measured based on the insulin administration instruction table 1005.
· Adding a record of the scheduled measurement date and time data of the patient identical to that of the collected/created data, if the scheduled measurement date and time has not been recorded in the patient history table 1006.
The details about these processes will be described later.

The incomplete patient warning processing section 1010 is activated by the scheduler 1007 at a predetermined time interval.
When finding a record that meets a predetermined condition in the patient history table 1006, the incomplete patient warning processing section 1010 will give a warning display of the patient and various information associated to the record. The details about this process will be described later.

Upon detecting the fact that the blood glucose meter 102 is mounted on the cradle 103, a blood glucose meter detection section 1012 activates a complete patient history processing section 1011 (Note: the term "complete" used herein and hereinafter means blood glucose is measured and/or insulin is administered).
The complete patient history processing section 1011 verifies whether or not there is any incomplete patient in a blood glucose measurement table downloaded from the blood glucose meter 102, and if yes, displays a warning on the display unit 907 to notify the verification result. The details about this process will be described later.
For the sake of simplicity, the description of the present embodiment is made based on a case where the patient's measurement data 117 is equal to the blood glucose measurement table, which is to be described later.

Further, upon detecting the fact that the blood glucose meter 102 is mounted on the cradle 103, the blood glucose meter detection section 1012 notifies such a fact to the display control section 1002.
Upon receiving such a notification, the display control section 1002 performs a display control, which is to be described later.

The program for realizing the function of the measurement data managing device 104 is formed by a plurality of programs.
The aforesaid incomplete patient history processing section 1009, incomplete patient warning processing section 1010 and complete patient history processing section 1011 are programs each operating independently. Since these programs operate at the same time, it is preferred that the OS is a multitasking OS.
Also, a single program having multi-thread function may be employed instead of the plurality of programs. In such a case, the OS may also be a single-tasking OS.
These programs or threads perform exclusive control with a known lock file mechanism or the like when performing write operation on various tables.

FIG. 11 shows an example of three tables provided inside the measurement data managing device 104. The three tables will be described below using sample data.
The patient information table 1004 is a table including a "PATIENT ID" field and a "PATIENT NAME" field, and is unique for each patient ID.

The insulin administration instruction table 1005 is a table for instructing times for performing blood glucose measurement and insulin administration for each patient from morning to night, and instructing prescription data for performing insulin administration. The insulin administration instruction table 1005 includes a "SERIAL NUMBER" field for uniquely identifying each record, the "PATIENT ID" field, a "SCHEDULED MEASUREMENT TIME" field, an "INSULIN THERAPY CLASSIFICATION" field and a "SCALE ID" field.
The reason why there are a plurality of the same patient IDs in the "PATIENT ID" field is because there are a plurality of times for performing blood glucose measurement and insulin administration for one patient.
Further, in order to be able to also cope with a case where a fixed amount of insulin is administered to the patient (referred to as "fixed-dose administration" hereinafter) regardless of the value of the blood glucose level, an "INSULIN THERAPY CLASSIFICATION" field formed by a flag variable is provided. The value of the "INSULIN THERAPY CLASSIFICATION" field includes logical "false" and logical "true", wherein the logical "false" means "performing insulin administration according to the scale ID", and the logical "true" means "administering a predetermined amount of predetermined insulin regardless the value of the measured blood glucose level".
The scale ID means prescription data. The prescription data is a table listing the insulin dosages to be administered corresponding to a range of the measured blood glucose level, and is also called a "sliding scale". There are a plurality of sliding scales prepared so as to be selected according to the state of the patient. When the "SCALE ID" field is blank, it means that only blood glucose measurement is to be performed, and insulin administration is not to be performed.

The patient history table 1006 is a table for accumulating data such as the blood glucose level of the patient measured by the blood glucose meter 102, the dosage of the administered insulin, and the like. In other words, the patient history table 1006 is a log. The patient history table 1006 includes the "PATIENT ID" field, a "SCHEDULED MEASUREMENT DATE AND TIME" field, the "SCALE ID" field, a "MEASUREMENT DATE AND TIME" field, a "BLOOD GLUCOSE LEVEL" field, an "INSULIN DOSAGE" field, an "INCOMPLETE FLAG" field and a "WARNING CONFIRMATION FLAG" field.
The "SCHEDULED MEASUREMENT DATE AND TIME" field is created based on the value of the "SCHEDULED MEASUREMENT TIME" field of the insulin administration instruction table 1005. In other words, the "SCHEDULED MEASUREMENT DATE AND TIME" is created by adding the current date to the value of the "SCHEDULED MEASUREMENT TIME" field.

Additional recording to the patient history table 1006 is conducted by the incomplete patient history processing section 1009 and the complete patient history processing section 1011 shown in FIG. 10. In the additional recording process conducted by the incomplete patient history processing section 1009, the following operations are performed.
· The "PATIENT ID" field, the "SCHEDULED MEASUREMENT DATE AND TIME" field and the "SCALE ID" field are filled.
· The "MEASUREMENT DATE AND TIME" field, the "BLOOD GLUCOSE LEVEL" field and the "INSULIN DOSAGE" field are left blank.
· The "INCOMPLETE FLAG" field is set to "true", and the "WARNING CONFIRMATION FLAG" field is set to "false".
   In the additional recording process conducted by the complete patient history processing section 1011, the following operations are performed.
· The "PATIENT ID" field, the "SCHEDULED MEASUREMENT DATE AND TIME" field and the "SCALE ID" field are filled.
· The "MEASUREMENT DATE AND TIME" field, the "BLOOD GLUCOSE LEVEL" field and the "INSULIN DOSAGE" field are also filled.
· The "INCOMPLETE FLAG" field is set to "false", and the "WARNING CONFIRMATION FLAG" field is also set to "false".

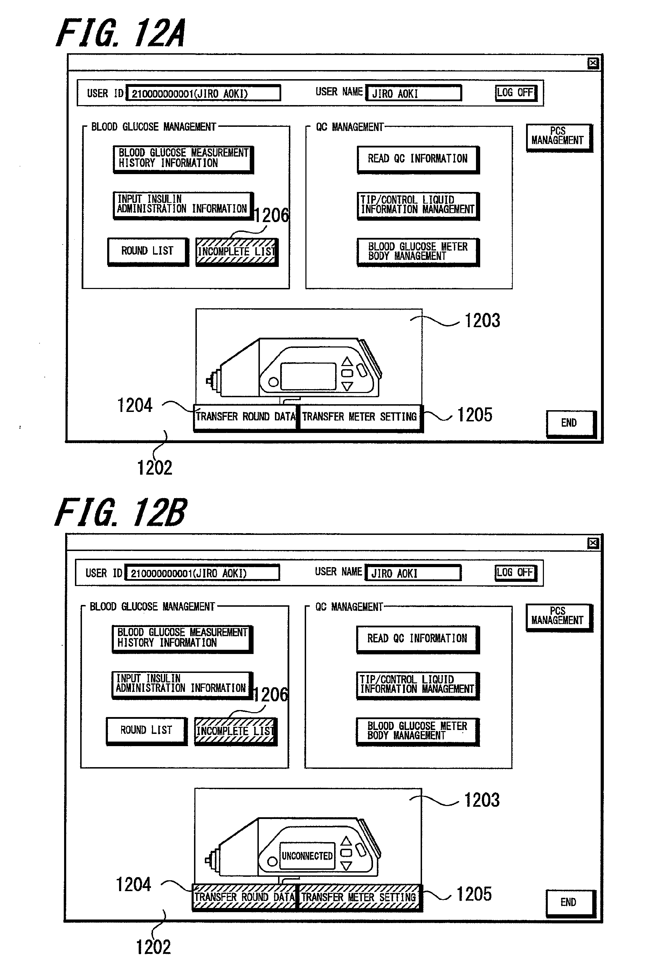

FIGS. 12A and 12B are views showing a top menu window 1202 displayed on the display unit of the measurement data managing device 104.
FIG. 12A shows the top menu window 1202 in a state where the blood glucose meter 102 is mounted on the cradle 103.
An illustration of the blood glucose meter 102 is displayed on a blood glucose meter state display column 1203.
A "TRANSFER ROUND DATA" button 1204 and a "TRANSFER METER SETTING" button 1205 are arranged immediately below the blood glucose meter state display column 1203.
The "TRANSFER ROUND DATA" button 1204 is a button for being pressed to transfer necessary data to the blood glucose meter for the blood glucose meter to perform blood glucose measurement for the patient.
The "TRANSFER METER SETTING" button 1205 is a button for being pressed to transfer various environmental settings of the blood glucose meter to the blood glucose meter.

FIG. 12B shows the top menu window 1202 in a state where the blood glucose meter 102 is removed from the cradle 103.
A character string "unconnected", which means the blood glucose meter 102 is not mounted, is displayed on the illustration of the blood glucose meter displayed in the blood glucose meter state display column 1203. At this time, the "TRANSFER ROUND DATA" button 1204 and the "TRANSFER METER SETTING" button 1205 are paled out and can not be pressed by a mouse or the like.
Further, an "INCOMPLETE LIST" button 1206 in an unpressable state is arranged immediately above the blood glucose meter state display column 1203.
The change of the blood glucose meter state display column 1203 is realized when the display control section 1002 receives a notification issued by the blood glucose meter detection section 1012.

### [Complete Patient History Processing Section]

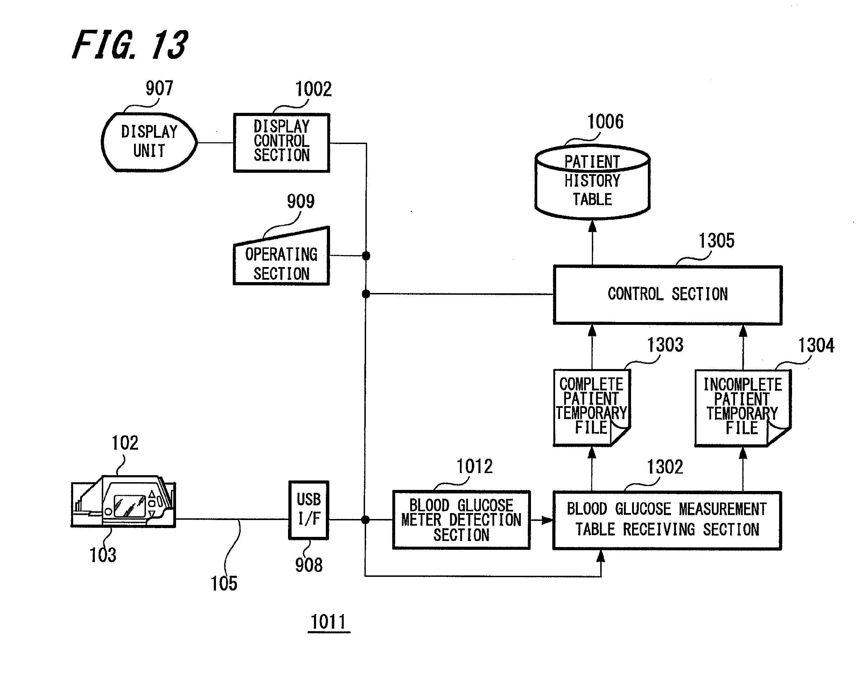

FIG. 13 is a functional block diagram of the complete patient history processing section 1011.
When the blood glucose meter 102 is mounted on the cradle 103, the blood glucose meter detection section 1012 detects the mounting state of the blood glucose meter 102 through the USB interface 908. The blood glucose meter detection section 1012 performs polling to the cradle 103 at a predetermined time interval.
Upon detecting the blood glucose meter 102, the blood glucose meter detection section 1012 activates the blood glucose measurement table receiving section 1302. Upon receiving the instruction of the blood glucose meter detection section 1012, the blood glucose measurement table receiving section 1302 downloads the blood glucose measurement table from the blood glucose meter 102. Further, the blood glucose measurement table receiving section 1302 divides the data of the downloaded blood glucose measurement table into data associated with blood-glucose-measured patient and data associated with blood-glucose-unmeasured patient, and respectively writes these data on a complete patient temporary file 1303 and an incomplete patient temporary file 1304.
After the blood glucose measurement table has been downloaded by the blood glucose measurement table receiving section 1302, the internal patient table 112, the user's basic data 115 and the tip lot data 116 stored in the blood glucose meter 102 are erased.

The complete patient temporary file 1303 and the incomplete patient temporary file 1304 are read into a control section 1305.
The control section 1305 records the content of the complete patient temporary file 1303 in the patient history table 1006. Further, the control section 1305 confirms whether or not the content of the incomplete patient temporary file 1304 is blank. As a result of the confirmation, if the content of the incomplete patient temporary file 1304 is not blank, i.e., if there is even one blood-glucose-unmeasured patient, the control section 1305 will display a warning on the display unit 907 through the display control section 1002. Further, the operating section 909 receives a selection made by the operator. In other words, when there is incomplete patient, the control section 1305 will query whether or not to record the data of the incomplete patient in the patient history table. As a result of the query, if the user has chosen to record the data of the incomplete patient, the control section 1305 will record the data of the incomplete patient in the patient history table. If the user has not chosen to record the data of the incomplete patient, the control section 1305 will read the patient ID and the scheduled measurement date and time data from the data of the incomplete patient temporary file 1304. Further, the control section 1305 searches the insulin administration instruction table 1005 using the read patient ID and scheduled measurement date and time data, creates data files necessary for performing blood glucose measurement such as the patient table, the prescription data and the like, and transmits the created data files to the blood glucose meter 102.

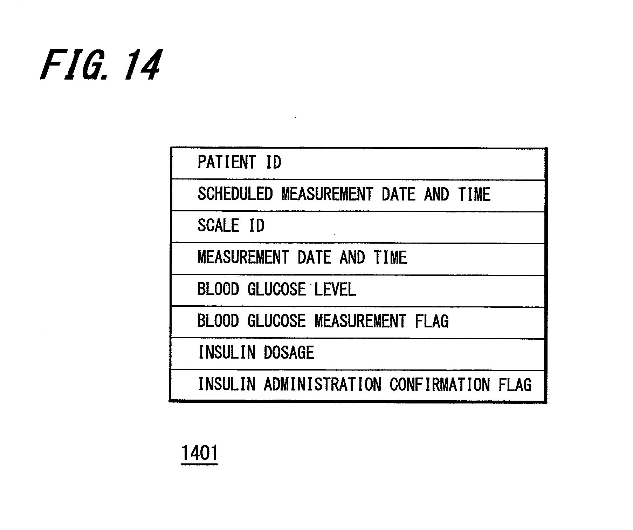

FIG. 14 shows fields of the blood glucose measurement table of the present embodiment received from the blood glucose meter 102.
When the blood glucose of the patient has been measured by the blood glucose meter 102, the "BLOOD GLUCOSE MEASUREMENT FLAG" field becomes "true".
When an input operation indicating "the insulin administration has been performed" is performed, "false" will be written into the "INSULIN ADMINISTRATION CONFIRMATION FLAG" field.
Incidentally, in the case of a patient whose "SCALE ID" field has no sliding scale ID recorded therein, since insulin administration is not performed, the insulin administration confirmation flag will be constantly "false".

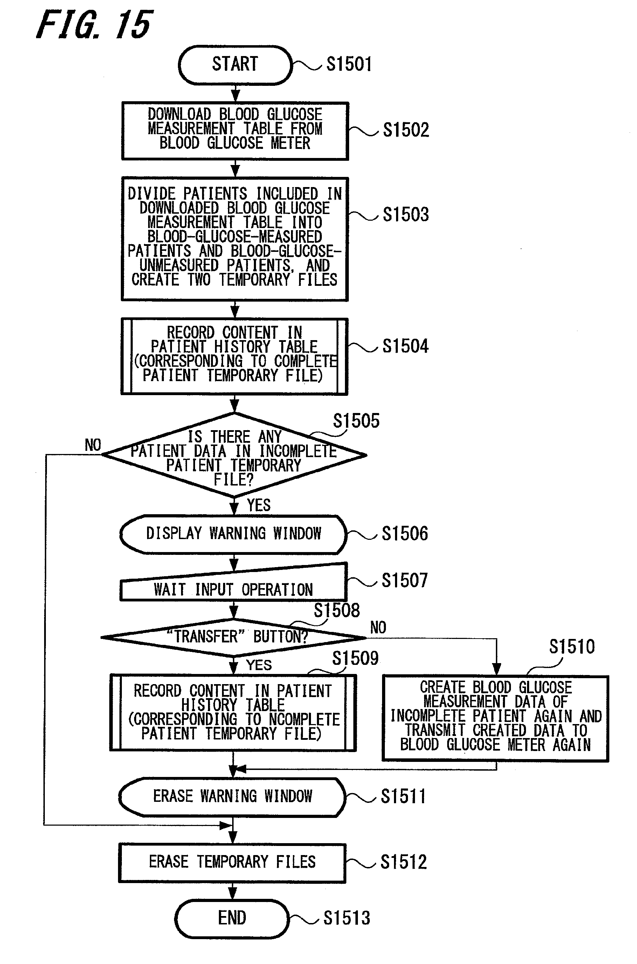

FIG. 15 is a flowchart showing the operation flow of the complete patient history processing section 1011.
Upon detecting the fact that the blood glucose meter 102 is mounted on the cradle 103 (Step S1501), the blood glucose meter detection section 1012 activates the blood glucose measurement table receiving section 1302. The blood glucose measurement table receiving section 1302 downloads a blood glucose measurement table 1401 from the blood glucose meter 102 (Step S1502). Further, the blood glucose measurement table receiving section 1302 divides the patients included in the downloaded blood glucose measurement table into the blood-glucose-measured patients and the blood-glucose-unmeasured patients, and creates two temporary files, which are the complete patient temporary file 1303 and the incomplete patient temporary file 1304 (Step S1503).
Next, the control section 1305 reads the complete patient temporary file 1303, and records read content in the patient history table 1006 (Step S1504).

Next, the control section 1305 reads the incomplete patient temporary file 1304, and confirms whether or not there is any patient data in the incomplete patient temporary file 1304 (Step S1505).
As a result of the confirmation, if there is no patient data in the incomplete patient temporary file 1304, i.e., if there is no blood-glucose-unmeasured patient ("NO" in Step S1505), both the complete patient temporary file 1303 and the incomplete patient temporary file 1304 will be erased (Step S1512), and the processing will be terminated (Step S1513).

As a result of the confirmation, if there is any patient data in the incomplete patient temporary file 1304, i.e., if there is any blood-glucose-unmeasured patient ("YES" in Step S1505), the control section 1305 first controls the display control section 1002 to display a warning window on the display unit 907 to report a warning to the user or the like (Step S1506). Further, the control section 1305 waits the operator to perform input operation from the operating section 909 (Step S1507).

If the button selected by the operating section (which is formed by a keyboard or a mouse) 909 is a "TRANSFER" button ("YES" in Step S1508), a process for recording the content of the incomplete patient temporary file 1304 in the patient history table 1006 will be performed (Step S1509).
If the button selected by the operating section (which is formed by a keyboard or a mouse) 909 is a "REMEASURE" button instead of the "TRANSFER" button ("NO" in Step S1508), the data files necessary for performing blood glucose measurement such as the patient table, the prescription data and the like will be created again based on the patient ID contained in data stored in the incomplete patient temporary file 1304, and the created data files will be transmitted again to the blood glucose meter (Step S1510).

The warning window will be erased if either the "TRANSFER" button or the "REMEASURE" button on the warning window is pressed (Step S1511). Further, both the complete patient temporary file 1303 and the incomplete patient temporary file 1304 are erased (Step S1512), and the processing is terminated (Step S1513).

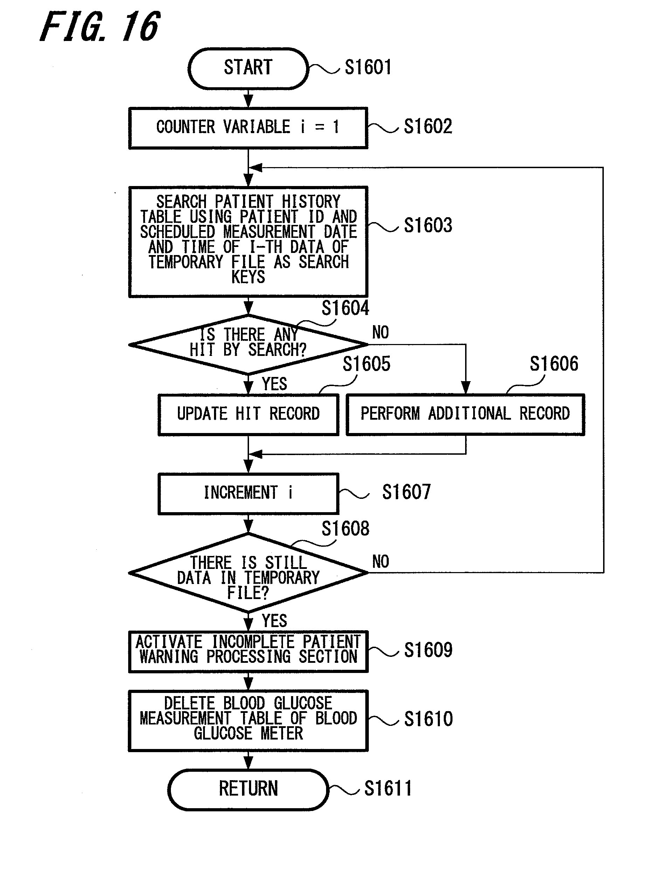

FIG. 16 is a flowchart showing the operation flow of the patient history table recording process. FIG. 16 shows the details of the processing of Steps S1504 and S1509 of FIG. 15.
When the processing is started (Step S1601), the control section 1305 first initializes an internal counter variable for performing a loop processing (which is to be described later) to one (Step S1602).

The following is loop processing.
The control section 1305 searches the patient history table 1006 using the patient ID and the value of the scheduled measurement date and time of the i-th data of the complete patient temporary file 1303 as search keys (Step S1603).
If there is any record in the patient history table 1006 hit by the search ("YES" in Step S1604), the control section 1305 will update the "MEASUREMENT DATE AND TIME" field, the "BLOOD GLUCOSE LEVEL" field and the "INSULIN DOSAGE" field of the hit record with the content of the i-th record of the complete patient temporary file 1303 (Step S1605).

At this time, the hit record is a record additionally recorded by an incomplete patient history processing section 1009 (which is to be described later), and the "INCOMPLETE FLAG" thereof is set to "true". In the update process of Step S1605, the "INCOMPLETE FLAG" is rewritten to "false". In other words, the record showing "blood glucose is unmeasured" is overwritten into the fact that "the blood glucose has been measured".

If there is no record hit by the search in Step S1603 ("NO" in Step S1604), the control section 1305 will additionally record the content of the i-th record of the complete patient temporary file 1303 in the patient history table 1006 as a new record (Step S1606). The content of the record is the following.
· The patient ID, the scheduled measurement date and time, the scale ID, and the scheduled measurement date and time.
· The "MEASUREMENT DATE AND TIME" field, the blood glucose level, and the insulin dosage.
· The "INCOMPLETE FLAG" is set to "false", and the "WARNING CONFIRMATION FLAG" is set to "false".

Further, the control section 1305 increments the counter variable i (Step S1607), and checks whether or not there is still any i-th data in the complete patient temporary file 1303 (Step S1608). If there is still any i-th data left in the complete patient temporary file 1303 ("NO" in Step S1608), the processing will be returned to Step S1603, and the processing will be repeated. If there is no i-th data left in the complete patient temporary file 1303 ("YES" in Step S1608), the incomplete patient warning processing section 1010 will be activated, the blood glucose measurement table 1401 of the blood glucose meter 102 will be deleted (Step S1610), and the processing will be terminated (Step S1611).

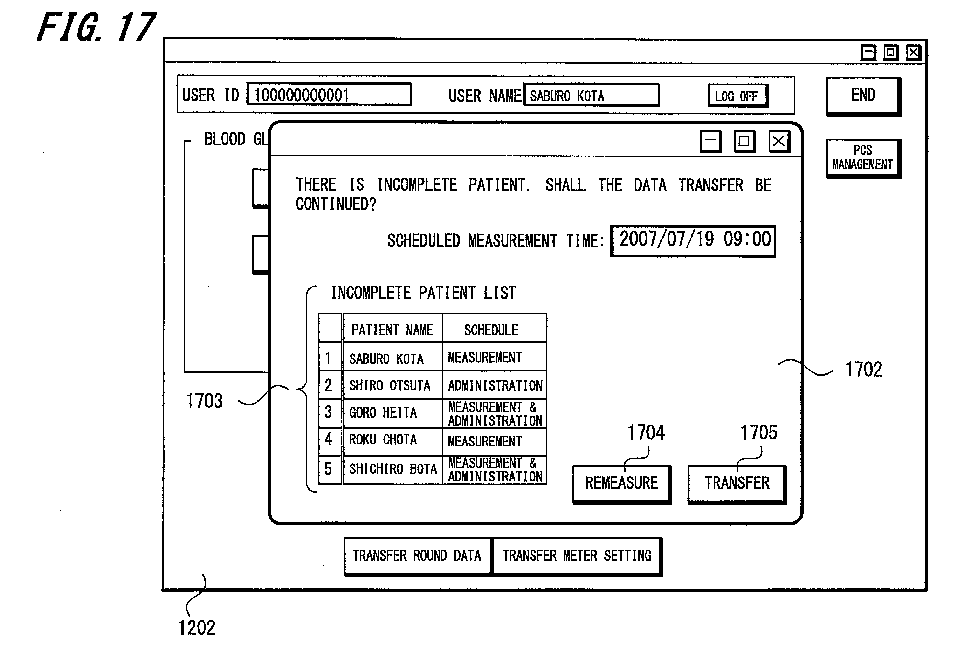

FIG. 17 is a schematic view showing a warning window 1702.
The warning window 1702 is overlaid on the top menu window 1202.
On the warning window 1702, an "INCOMPLETE PATIENT LIST" display column 1703 is display, and a "REMEASURE" button 1704 and a "TRANSFER" button 1705 are provided.
The "INCOMPLETE PATIENT LIST" display column 1703 is created by reading the complete patient temporary file 1303 with the control section 1305.
When the "REMEASURE" button 1704 is pressed, the control section 1305 will record the data of the blood-glucose-measured patient in the patient history table 1006, and, as for incomplete patient, create the data files necessary for performing blood glucose measurement such as the patient table, the prescription data and the like again, and transmit the created data files to the blood glucose meter 102. Thereafter, the warning window 1702 is erased. The person in charge of the measurement picks up the blood glucose meter 102 from the cradle 103 again to perform blood glucose measurement or insulin administration on the incomplete patient.
When the "TRANSFER" button 1705 is pressed, the control section 1305 will record the received blood glucose measurement table 1401 in the patient history table 1006, delete the internal patient table 112 as well as the user's basic data 115 and the tip lot data 116, and then erase the warning window 1702.

During the blood glucose measurement operation, it is difficult for the blood glucose meter single body to confirm the existence of the patient whose blood glucose is forgotten to be measured.
On the other hand, the timing when the blood glucose measurement table 1401 is transferred to the measurement data managing device 104 after completing the blood glucose measurement operation is the best timing to check the existence of the patient whose blood glucose is forgotten to be measured.
Therefore, the blood glucose measurement flag is confirmed once the blood glucose measurement table 1401 has been downloaded. The warning window 1702 will be displayed if it is confirmed that there is even one patient whose blood glucose has not been measured. At this time, the internal patient table 112 and the like in the blood glucose meter will be deleted, and if the nurse really has forgotten to perform blood glucose measurement and/or insulin administration, the nurse will download the patient data again and perform blood glucose measurement, insulin administration and/or the like on the patient.
In such a manner, the error of forgetting to measure the blood glucose can be prevented by the system according to the present embodiment.

### [Incomplete Patient History Processing Section]

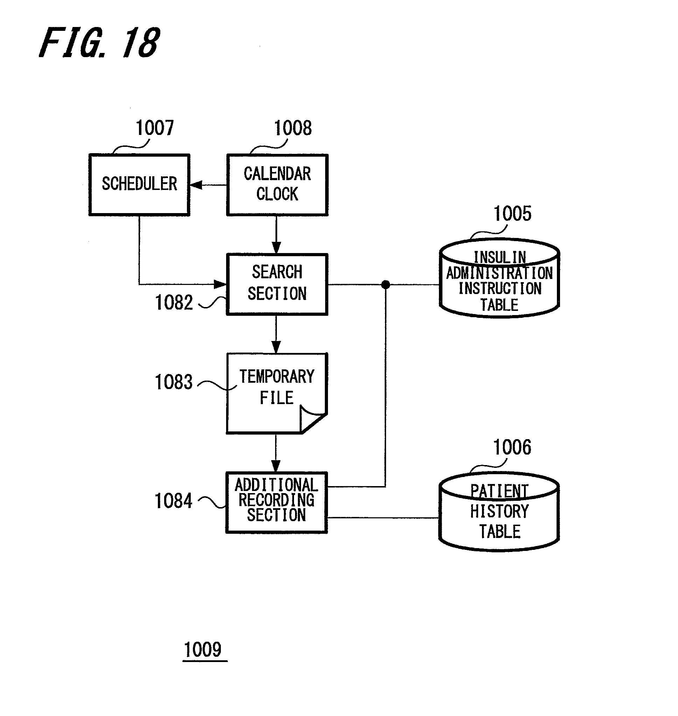

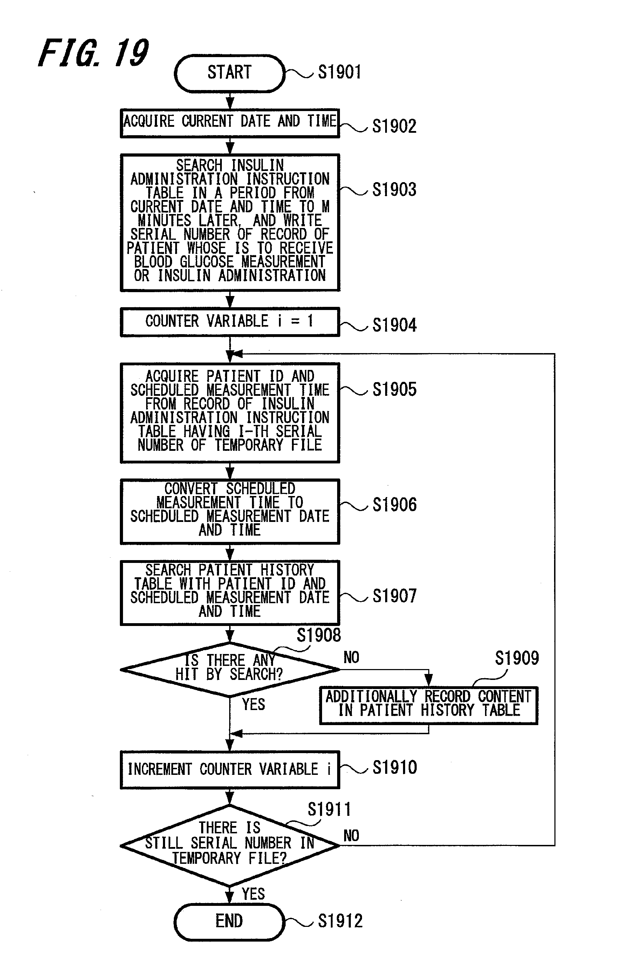

The operation of the incomplete patient history processing section 1009 will be described below with reference to FIGS. 18 and 19.
FIG. 18 is a functional block diagram showing the incomplete patient history processing section 1009.
FIG. 19 is a flowchart showing the operation flow of the incomplete patient history processing section 1009.
A search section 1802 is activated by the scheduler 1007 adapted to monitor the date and time information of the calendar clock 1008 (Step S1901).
Upon being activated by the scheduler 1007, the search section 1802 acquires the date and time information from the calendar clock 1008 (Step S1902), and searches for the scheduled measurement time in the insulin administration instruction table 1005 in a period from current date and time to m minutes later (Step S1903). Incidentally, m can be set to any value. For example, m can be set to 60.
The search result is recorded in a temporary file 1803. Only the serial numbers of insulin administration instruction table 1005 are listed in the temporary file 1803.

An additional recording section 1804 is activated in response to the existence of the temporary file 1803.
The additional recording section 1804 first initializes the internal counter variable i to one (Step S1904).
The following is a loop processing.
The additional recording section 1804 searches the insulin administration instruction table 1005 with the serial number in the temporary file 1803. Further, the additional recording section 1804 acquires the patient ID and the scheduled measurement time from the record hit by the search (Step S1905). After converting the scheduled measurement time to the scheduled measurement date and time (Step S1906), the additional recording section 1804 searches the patient history table 1006 using the scheduled measurement date and time and the patient ID as the search keys (Step S1907).

If there is no record hit by the search ("NO" in Step S1908), the additional recording section 1804 will additionally records the following content in the patient history table 1006 (Step S1909).
· The content of the record of the insulin administration instruction table 1005, which is the basis of the search keys.
· Converting the scheduled measurement time into the scheduled measurement date and time.
· Setting the "INCOMPLETE FLAG" to "true", and setting the "WARNING CONFIRMATION FLAG" to "false".
If there is any record hit by the search ("YES" in Step S1908), the additional recording section 1804 will not perform any operation.
Further, the search section 1802 increments the counter variable i (Step S1910), and confirms the existence of the i-th serial number in the temporary file 1803 (Step S1911). If there is the i-th serial number in the temporary file 1803 ("NO" in Step S1911), the processing will be repeated (Step S1905). While if there is no the i-th serial number in the temporary file 1803 ("YES" in Step S1911), the processing will be terminated (Step S1912).

The incomplete patient history processing section 1009 is adapted to record the "blood glucose is unmeasured" histories in the patient history table 1006.
In contrast, the complete patient history processing section 1011 is adapted to record the "blood glucose is measured" histories in the patient history table 1006.
Either the processing of the incomplete patient history processing section 1009 or the processing of the complete patient history processing section 1011 of the same patient ID at the same scheduled measurement date and time is first additionally recorded in the patient history table 1006.

Therefore, in order to prevent incorrect recording, the incomplete patient history processing section 1009 confirms whether or not there is any record previously recorded in the patient history table 1006. In other words, the incomplete patient history processing section 1009 first checks whether or not there is any record recorded by the complete patient history processing section 1011. If there is any record previously recorded, the incomplete patient history processing section 1009 will not perform additional recording operation. While if there is no record previously recorded, the incomplete patient history processing section 1009 will perform additional recording operation. As is to be described later, the additionally recorded records are items to be listed in the incomplete patient warning processing section 1010.

The record recorded by the complete patient history processing section 1011 precedes the record additionally recorded by the incomplete patient history processing section 1009.
Even after m minutes has elapsed from the scheduled blood glucose measurement time and a warning has been issued by the incomplete patient warning processing section 1010, when the person in charge of the measurement, such as a nurse, mounts the blood glucose meter 102 on the cradle 103, the measurement data managing device 104 will download the blood glucose measurement table 1401 from the blood glucose meter 102.
From the downloaded blood glucose measurement table 1401, the complete patient history processing section 1011 records the fact that "the blood glucose measurement has been performed" in the patient history table 1006.
At this time, the record of "blood glucose is unmeasured" additionally recorded by the incomplete patient history processing section 1009 shall be overwritten.
The complete patient history processing section 1011 searches the patient history table 1006 to check whether or not there is any record additionally recorded by the incomplete patient history processing section 1009. If there is such a record, the complete patient history processing section 1011 records the data in the blank field, and rewrites the "true" of the incomplete flag, which is written by the incomplete patient history processing section 1009, into the "false". While there is no such record, the complete patient history processing section 1011 performs additional recording operation.

### [Incomplete Patient Warning Processing Section]

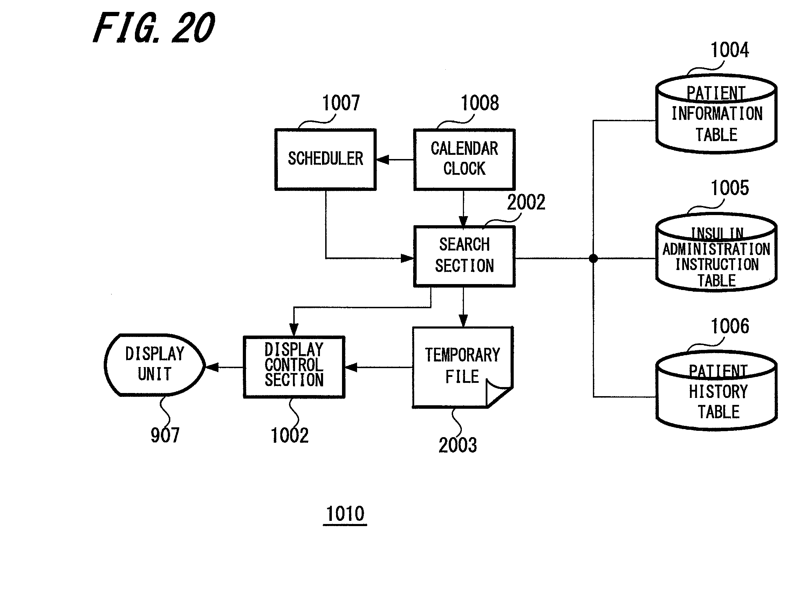

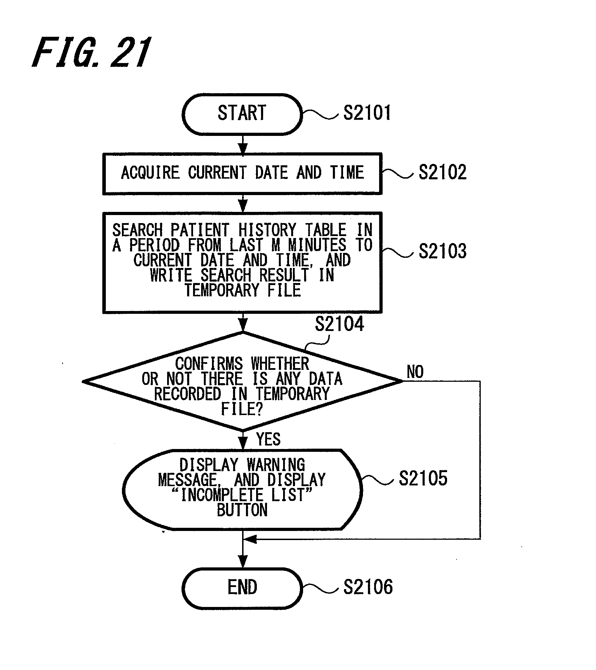

The operation of the incomplete patient warning processing section 1010 will be described below with reference to FIGS. 20 and 21.
FIG. 20 is a functional block diagram of the incomplete patient warning processing section 1010.
FIG. 21 is a flowchart showing the operation flow of the incomplete patient warning processing section 1010.
The search section 1802 is activated by the scheduler 1007 adapted to monitor the date and time information of the calendar clock 1008 (Step S2101).
Upon being activated by the scheduler 1007, the search section 2002 acquires the date and time information from the calendar clock 1008 (Step S2101), and searches for the record in the patient history table 1006 having an incomplete flag "true" and a warning confirmation flag "false" in a period from the last m minutes to current date and time (Step S2103). Incidentally, m can be set to any value. For example, m can be set to 60.

The search result is recorded in a temporary file 2003. The patient ID, the scheduled measurement date and time, and the incomplete flag is listed in the temporary file 2003.
After creating the temporary file, the search section 2002 confirms whether or not there is any data recorded in the temporary file 2003 (Step S2104). If there is any data in the temporary file 2003, the search section 2002 will control the display control section 1002 to display a warning message in the top menu window 1202 displayed on the display unit 907. Further, the "INCOMPLETE LIST" button 1206 is validated so as to be pressable (Step S2105), and the processing is terminated (Step S2106).

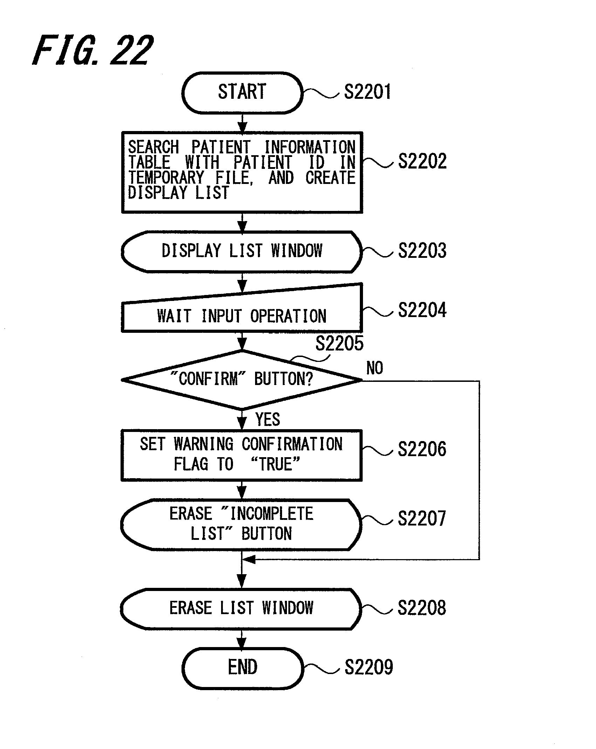

FIG. 22 is a flowchart showing the operation flow of the incomplete list display process. Such a process is an internal process of the data edit processing section 1003.
When the "INCOMPLETE LIST" button 1206 is pressed by the operator (Step S2201), the data edit processing section 1003 first searches the patient information table 1004 with the patient ID in the temporary file 2003 created by the search section 2002 and creates a display list (Step S2202).
Next, the data edit processing section 1003 controls the display control section 1002 based on the display list to display an incomplete patient list window on the display unit 907 (Step S2203). Further, the buttons in the incomplete patient list window are waited to be operated (Step S2204).
If a "CONFIRM" button is pressed ("YES" in Step S2205), the warning confirmation flag in the record of the patient history table 1006 will be rewritten into "true" (Step S2206), wherein the warning confirmation flag corresponds to the patient ID and the scheduled measurement date and time existing in the temporary file 2003. Further, the "INCOMPLETE LIST" button 1206 in the top menu window 1202 is invalidated and erased (Step S2207).
While if a "RETURN" button is pressed ("NO" in Step S2205), no operation will be performed.
Further, the incomplete patient list window is erased (Step S2208), and the processing is terminated (Step S2209).

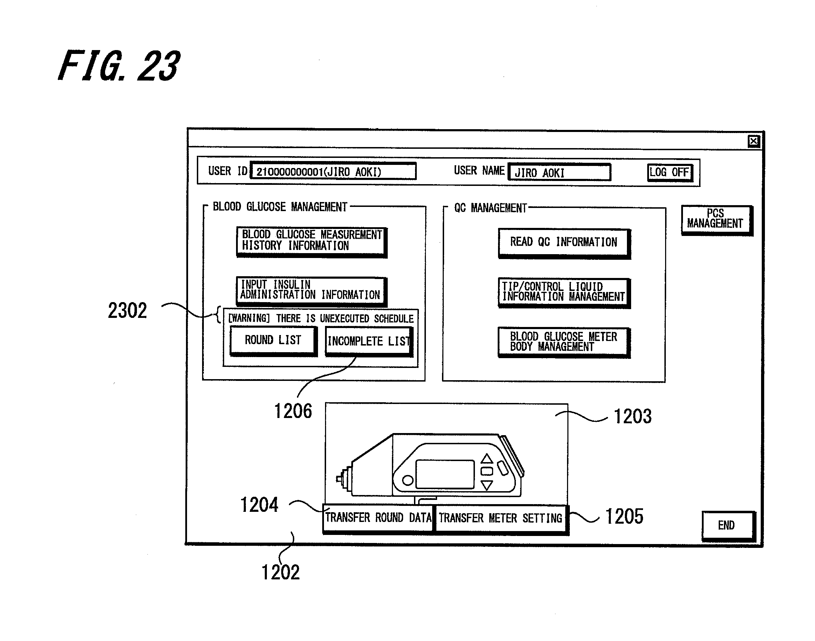

FIG. 23 is a view showing the state of the top menu window after a predetermined time has elapsed from the scheduled blood glucose measurement time.
The "INCOMPLETE LIST" button 1206 is clearly displayed, and is in a pressable state.
A warning message 2302 is displayed immediately above the "INCOMPLETE LIST" button 1206.

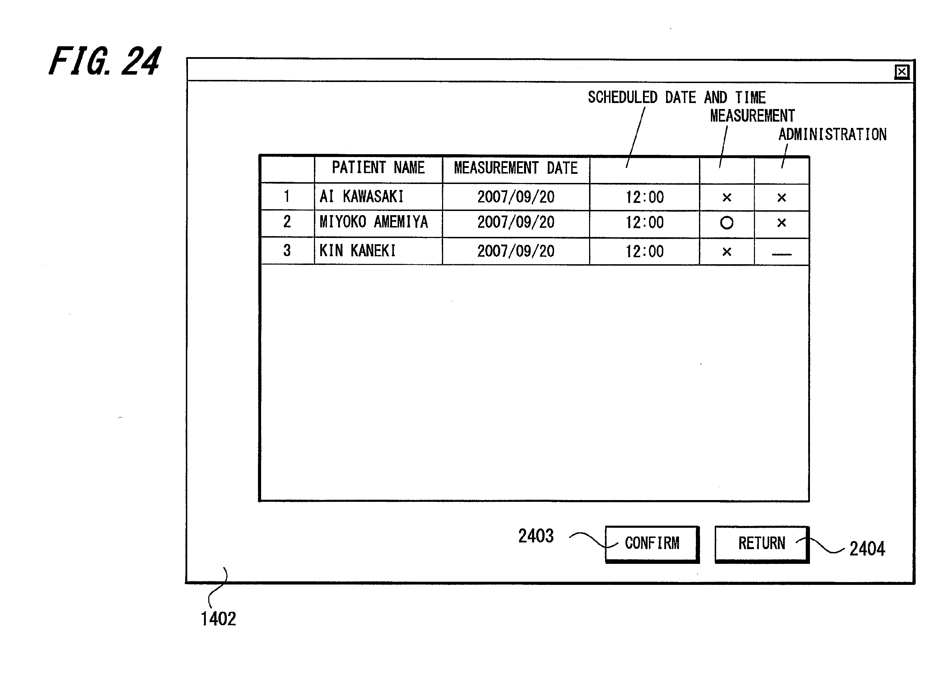

FIG. 24 is a view showing the incomplete patient list window.
The patients whose blood glucose is forgotten to be measured and/or whose insulin is forgotten to be administered are listed in an incomplete patient list window 2402 from the top.
If a "CONFIRM" button 2403 is pressed, the warning confirmation flag in the record of the patient history table 1006 will be rewritten into "true", wherein the warning confirmation flag corresponds to the patient ID and the scheduled measurement date and time existing in the temporary file 2003. Further, the "INCOMPLETE LIST" button 1206 is erased, and the incomplete patient list window 2402 is erased. Thus, the warning message is not displayed, and the "INCOMPLETE LIST" button 1206 becomes unpressable.
While if a "RETURN" button 2404 is pressed, the incomplete patient list window 2402 will be erased without doing anything to the patient history table 1006. Thus, the warning message stays displayed, and the "INCOMPLETE LIST" button 1206 is in pressable state.

The blood glucose measuring system is disclosed in the present embodiment.
The incomplete patient history processing section periodically searches the insulin administration instruction table in which the scheduled measurement times are listed for each patient, and temporarily creates a list of the patients not subjected to blood glucose measurement and/or the like. Thereafter, the incomplete patient history processing section searches the patient history table to see whether or not there is any record, and then sets the incomplete flag to "true" and additionally records a new record. In other words, the incomplete patient history processing section records the fact that "blood glucose measurement and/or the like is not performed" in the patient history table.
The operation for the incomplete patient warning processing section to display a warning on the display unit can be performed by searching for the incomplete flag in the patient history table. Such an operation can be performed asynchronously with the incomplete patient history processing section.

The complete patient history processing section 1011 downloads the blood glucose measurement table 1401 from the blood glucose meter, and once stores the downloaded blood glucose measurement table 1401 in the temporary file. The complete patient history processing section 1011 searches the records in the patient history table one by one to see whether or not there is any record identical to the records in the temporary file already having been additionally recorded by the incomplete patient history processing section. If there is any record identical to the records in the temporary file, the incomplete flag will be rewritten to "false", and the measurement data will be recorded in the blank field. In other words, the records representing "incomplete" are overwritten with the fact that "the measurement has been performed" and the measurement data.
These processes are independently performed, therefore it is possible to preferentially record the fact that "the measurement has been performed" in the patient history table, and at the same time, if there is a possibility that the measurement might be forgotten to be performed, the incomplete patient warning processing section will securely display a warning. Owing to such a process, accident of forgetting to perform measurement can be prevented within a required time interval.

Further, the complete patient history processing section checks the blood glucose measurement table 1401, which is downloaded from the blood glucose meter and stored in the temporary file, and, if finding there is any blood-glucose-unmeasured patient, displays the warning window to query whether the data shall be received as it is, or whether the measurement shall be performed on the patient whose blood glucose is forgotten to be measured. In other words, accident of forgetting to perform measurement can be known as soon as the blood glucose meter is mounted on the cradle. This function can not be achieved by the blood glucose meter single body.

### [Second Embodiment]

### [Blood Glucose Measurement Processing of Blood Glucose Meter 102]

The flow of blood glucose measurement procedure with the blood glucose meter 102 will be described below with reference to FIG. 25.
FIG. 25 is a view schematically showing the flow of blood glucose measurement procedure with the blood glucose meter 102.
(1) A patient ID 2503, which is a bar-code indicated in a name tag or the like of a patient 2502, is read by the bar-code reader 208.
The read patient ID 2503 is first used a search key for searching the internal patient table 112.
The patient data 114 previously transmitted from the measurement data managing device 104 is converted into the internal patient table 112 inside the blood glucose meter 102, and the result is stored in the nonvolatile storage 614. The internal patient table 112 is searched with the patient ID 2503. The record hit by the search is the record of the patient in the internal patient table 112, in which various data are to be recorded.

(2) A user ID 2505, which is a bar-code indicated in a name tag or the like of a nurse 2504, is read by the bar-code reader 208.
Verification is performed to see whether or not the read user ID is included in the user's basic data 115. The user's basic data 115 is searched using the user ID as a search key, and if there is the user ID, a "USER ID" field of the previously identified record in the internal patient table 112 will be overwritten by the user ID.

(3) A tip lot number 2507, which is a bar-code printed in a box 2506 of the measuring tip 212, is read by the bar-code reader 208.
Verification is performed to see whether or not the read tip lot number 2507 is included in the tip lot data 116 (see FIG. 1). The tip lot data 116 is searched using the tip lot number as a search key, and if there is the tip lot number, a "TIP LOT NUMBER" field of the previously identified record in the internal patient table 112 will be overwritten by the tip lot number.

(4) Immediately after recording the tip lot number in the "TIP LOT NUMBER" field of the internal patient table 112 in (3), the ambient temperature is measured by the thermistor 606. Further, if it is judged that the ambient temperature is in a predetermined range, a "TEMPERATURE AT MEASUREMENT TIME" field of the previously identified record in the internal patient table 112 will be overwritten by the ambient temperature.

(5) The measuring tip 212 is attached to the optical measuring section of the blood glucose meter 102 to measure the blood glucose. Further, the date and time data at measurement time is obtained from the calendar clock 607.
The "BLOOD GLUCOSE LEVEL" field of the previously identified record in the internal patient table 112 is overwritten by the measured blood glucose level. The "MEASUREMENT DATE AND TIME" field of the previously identified record in the internal patient table 112 is overwritten by the date and time data.
A flag representing the fact that "the blood glucose has been measured" is recorded in a "MEASUREMENT/DISPLAY/ADMINISTRATION FLAG" field.
Thereafter, the measured blood glucose level is displayed on the LCD 203 (the display unit 615).

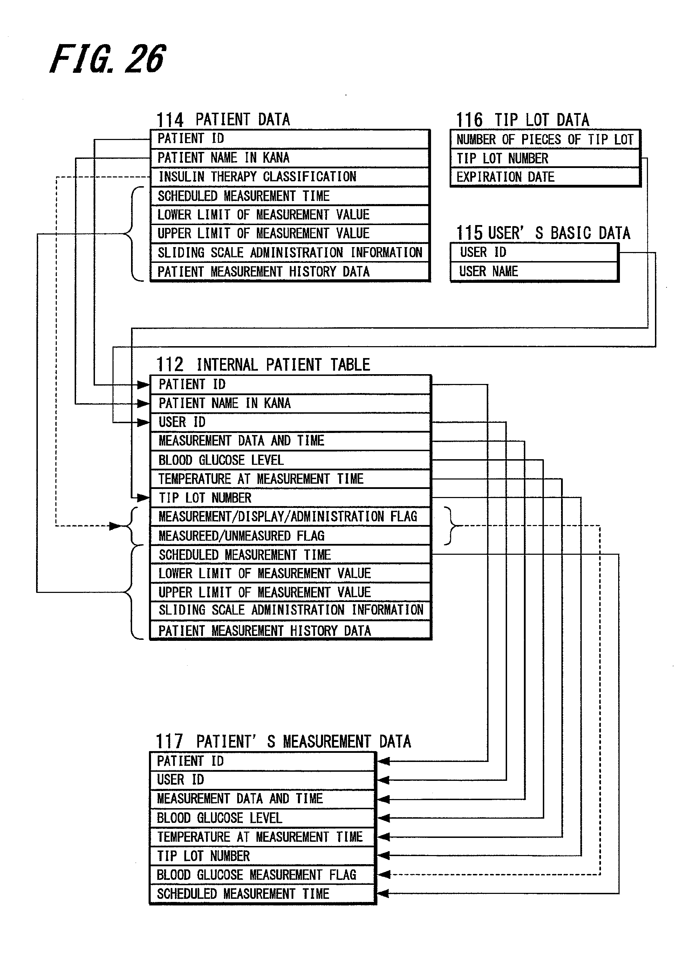

(6) A sliding scale 2509 of the patient 2502 is searched with the measured blood glucose level, and kind and dosage of the drug (such as insulin and/or the like) prescribed for the patient 2502 are displayed on the LCD 203. The sliding scale 2509 is stored in the internal patient table 112 for each patient ID. The details about the sliding scale 2509 will be described later with reference to FIG. 26.
(7) Following the prescription displayed on the LCD 203, the nurse 2504 administers the insulin and/or the like with a syringe 2508, and then inputs such a fact with the Enter key 206. As a result, a flag representing the fact that "prescription has been performed" is recorded in the "MEASUREMENT/DISPLAY/ADMINISTRATION FLAG" field of the internal patient table 112.

By performing the aforesaid measurement operation, the following contents are recorded in the internal patient table 112:
· Which user
· has (or has not) measured blood glucose
· for which patient
· with respect to what scheduled measurement time
· using a tip of what tip lot number
· at what ambient temperature
· What is the blood glucose level (if blood glucose has been measured)
· What is the measurement date and time (if blood glucose has been measured)
· Whether or not insulin and/or the like has been prescribed.

The blood glucose measurement operation and the insulin prescription operation are generally performed at a predetermined time after the patient having meal. Further, the blood glucose measurement procedure and the insulin prescription procedure may also be performed at a predetermined time before the patient having meal. The blood glucose measurement operation and the insulin prescription operation are collectively performed for a plurality of patients at a predetermined time.
The work unit of the blood glucose measurement operation and/or the insulin prescription operation collectively performed for a plurality of patients at a predetermined time is "round". For example, the expression of "one round in 30 minutes after breakfast" and the like are used in practice.
In order to prevent mistake in the blood glucose measurement operation, the insulin prescription operation and the like, the measurement data managing device 104 only transmits data for performing one round to the blood glucose meter 102. Such data are the patient data 114, the user's basic data 115 and the tip lot data 116.
Further, make sure that the blood glucose meter 102 is mounted on the cradle 103 after the round is completed. As soon as the blood glucose meter 102 is mounted on the cradle 103, the patient's measurement data 117 is created by extracting predetermined fields of the internal patient table 112, and then the created patient's measurement data 117 is transmitted from the blood glucose meter 102 to the measurement data managing device 104. Upon receiving the patient's measurement data 117, the measurement data managing device 104 records the patient's measurement data 117 in the patient history table 1006 inside thereof.

FIG. 26 is a view showing internal constitution of each of the tables and correlation between the tables. FIG. 26 is a diagram called an "ER diagram" (ER is an abbreviation for Entity-Relationship). Incidentally, FIG. 26 is created based on correlation between the tables transmitted and received between the measurement data managing device 104 and the blood glucose meter 102 shown in FIG. 1.
All fields (except for the "INSULIN THERAPY CLASSIFICATION" field) of the patient data 114 transmitted from the measurement data managing device 104 are recorded in the internal patient table 112 as they are. The internal patient table 112 is stored in the nonvolatile storage 614 inside the blood glucose meter 102.
The "INSULIN THERAPY CLASSIFICATION" field of the patient data 114 is recorded in the "MEASUREMENT/DISPLAY/ADMINISTRATION FLAG" field and a "MEASURED/UNMEASURED FLAG" field of the internal patient table 112 after being subjected to a predetermined conversion processing.
The tip lot data 116 and the user's basic data 115 transmitted from the measurement data managing device 104 is stored in the nonvolatile storage 614 inside the blood glucose meter 102 as they are. Further, the items (the tip lot number and the user ID) read by the bar-code reader 208 during the blood glucose measurement operation are recorded in the internal patient table 112 every time they are read.

There exists a case where two or more kinds of drugs are administered to the patient. In such a case, although not shown in FIG. 26, the kind of the drugs and the insulin administration confirmation flag for each of the drugs are arranged in the internal patient table. In the case where a plurality of drugs are administered to a patient, the result of the "MEASUREMENT/DISPLAY/ADMINISTRATION FLAG" field is recorded for each of the drugs.

### [Patient Data 114]

Next, each of the fields of the patient data 114 will be described below.
Any one of the following cases is recorded in the "INSULIN THERAPY CLASSIFICATION" field of the patient data 114:
0: Performing blood glucose measurement only.
1: Performing "blood glucose measurement" and "insulin administration using sliding scale".
2: Performing "blood glucose measurement" and "insulin administration not using sliding scale (referred to as "fixed-dose administration" hereinafter)".
5: Performing insulin administration using sliding scale only (without performing blood glucose measurement).
6: Performing fixed-dose insulin administration only (without performing blood glucose measurement).

The sliding scale means prescription data. The sliding scale is a table listing the dosages of the insulin and/or like to be administered corresponding to a range of the measured blood glucose level.
"Fixed-dose administration" means administering a fixed amount of insulin and/or the like regardless of the measuring result of the blood glucose level.
The sliding scale is stored in a "SLIDING SCALE ADMINISTRATION INFORMATION" field of the patient data 114.

The value of the "INSULIN THERAPY CLASSIFICATION" field of the patient data 114 represents flag information for instructing the blood glucose meter 102 what operation should be performed on each of the patients. For example:
· Value "0" means that blood glucose measurement only should be performed on the patient.
· Value "1" means that "blood glucose measurement" and "insulin administration using sliding scale" should be performed on the patient.
The same goes for "2", "5" and "6".

As shown in FIG. 25, the "PATIENT ID" field is a number for identifying the patient, and is recorded in the bar-code attached to the clothes of the patient 2502. The patient name in Japanese kana is indicated in a "PATIENT NAME IN KANA" field. The patient name in Japanese kana as well as the patient ID are displayed on the LCD 203 of the blood glucose meter 102 for the user to correctly identify the patient in his(or her) side.
The "SCHEDULED MEASUREMENT TIME" field represents the scheduled time for performing round, and also is used as a search key for identifying data on side of the measurement data managing device 104.
A "LOWER LIMIT OF MEASUREMENT VALUE" field and an "UPPER LIMIT OF MEASUREMENT VALUE" field represent a range of blood glucose level capable of performing insulin administration. The "LOWER LIMIT OF MEASUREMENT VALUE" field and "UPPER LIMIT OF MEASUREMENT VALUE" are provided for preventing accident caused by improper insulin administration, as well as for indirectly knowing patient's condition, especially in the case where the value of the "INSULIN THERAPY CLASSIFICATION" field is "1" or "2". In other words, if the blood glucose level is not in the aforesaid range of blood glucose level, it can be judged that patient's condition is not good, and therefore it is not proper to perform insulin administration on the patient.
A "PATIENT MEASUREMENT HISTORY DATA" field is adapted to store a plurality of latest blood glucose measurement history information of the patient. Further, when the value of the "INSULIN THERAPY CLASSIFICATION" field is "5", the value of the blood glucose level last measured is stored, and insulin administration using sliding scale is performed based on this value.
The above is the description of each of the fields of the patient data 114.

### [Internal Patient Table 112]

Next, each of the fields of the internal patient table 112 will be described below.
The "MEASUREMENT/DISPLAY/ADMINISTRATION FLAG" field is adapted to store the "blood glucose measurement flag", the "insulin administration confirmation flag" and an "insulin dosage display flag".
Any one of the following values is recorded in the "MEASURED/UNMEASURED FLAG" field:
· "1", which represents logical "true", when the "INSULIN THERAPY CLASSIFICATION" field of the patient data 114 is "0", "1" or "2".
· "0", which represents logical "false", when the "INSULIN THERAPY CLASSIFICATION" field of the patient data 114 is "5" or "6".
The "MEASUREMENT/DISPLAY/ADMINISTRATION FLAG" field changes every time when: (1) immediately after blood glucose measurement operation has been completed; (2) insulin dosage has been displayed; and (3) after inputting the fact that "insulin administration has been performed".
The contents of the "MEASUREMENT/DISPLAY/ADMINISTRATION FLAG" field and the "MEASURED/UNMEASURED FLAG" field are reflected in "BLOOD GLUCOSE MEASUREMENT FLAG" field of the patient's measurement data 117.
Incidentally, details about the change of these flags will be described later.

The "PATIENT ID" field, the "PATIENT NAME IN KANA" field, the "SCHEDULED MEASUREMENT TIME" field, the "LOWER LIMIT OF MEASUREMENT VALUE" field, the "UPPER LIMIT OF MEASUREMENT VALUE" field, the "SLIDING SCALE ADMINISTRATION INFORMATION" field and the "PATIENT MEASUREMENT HISTORY DATA" field are copied in their entirety from the patient data 114.
The user ID read by the bar-code reader 208 is recorded in the "USER ID" field after being compared by the tip lot data 116.
The tip lot number read by the bar-code reader 208 is recorded in the "TIP LOT NUMBER" field after being compared by the user's basic data 115.
As is described with reference to FIG. 25, the value of each of the "MEASUREMENT DATE AND TIME" field, the "BLOOD GLUCOSE LEVEL" field and the "TEMPERATURE AT MEASUREMENT TIME" field is recorded every time the blood glucose measurement is performed with the blood glucose meter 102.
The above is the description of each of the fields of the internal patient table 112.

### [Patient's Measurement Data 117]

In the internal patient table 112, the "PATIENT ID" field, the "USER ID" field, the "MEASUREMENT DATE AND TIME" field, the "BLOOD GLUCOSE LEVEL" field, the "TEMPERATURE AT MEASUREMENT TIME" field, the "TIP LOT NUMBER" field and the "SCHEDULED MEASUREMENT TIME" field, which are necessary for the measurement data managing device 104, are outputted in their entirety as the patient's measurement data 117.
The contents of the "MEASUREMENT/DISPLAY/ADMINISTRATION FLAG" field and the "MEASURED/UNMEASURED FLAG" field are outputted as the data of the "BLOOD GLUCOSE MEASUREMENT FLAG" field after being subjected to a predetermined conversion processing.
The above is the description of each of the fields of the patient's measurement data 117.

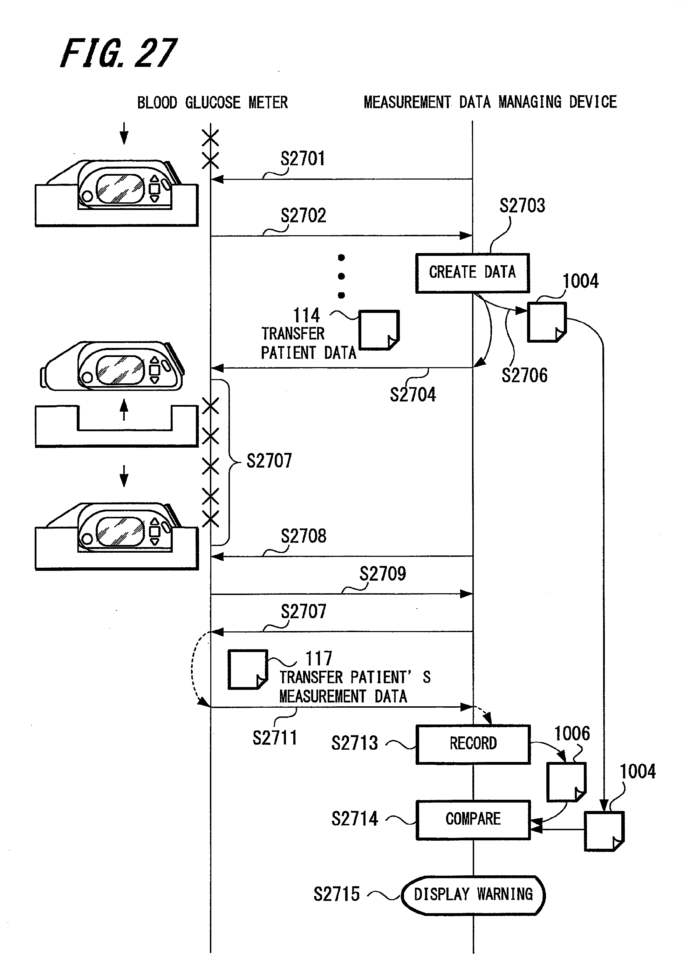

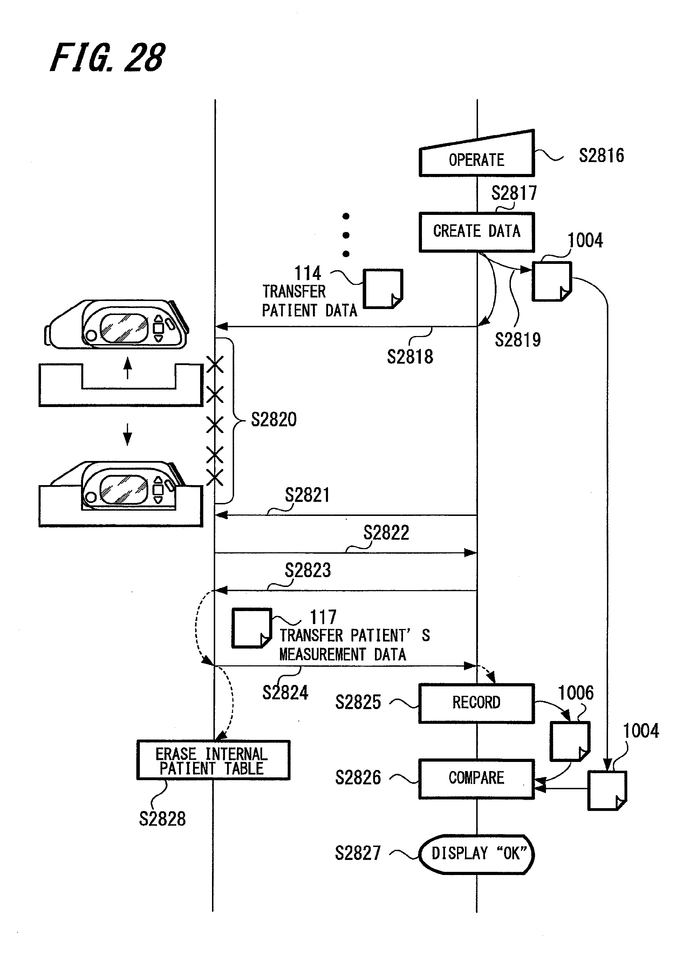

FIGS. 27 and 28 are flowcharts showing the flow of the communication between the blood glucose meter 102 and the measurement data managing device 104.
The measurement data managing device 104 constantly performs polling to the cradle 103 (Step S2701). In such a state, when the blood glucose meter 102 is mounted on the cradle 103, the blood glucose meter 102 responds to the polling command sent from the measurement data managing device 104. The measurement data managing device 104 receives the response of the blood glucose meter 102, and thereby immediately identifies the existence of the blood glucose meter 102. The operation of establishing communication between the blood glucose meter 102 and the measurement data managing device 104 is called "negotiation" (Step S2702).
The user (such as a nurse or the like) operates the measurement data managing device 104 to create data for performing round (Step S2703). Further, when "transfer" is instructed, the patient data 114, the user's basic data 115 and the tip lot data 116 are transmitted to the blood glucose meter 102 (Step S2704). At this time, a part of the content of the patient data 114 is written in the patient information table 1004 inside the measurement data managing device 104 (Step S2706).

The user picks up the blood glucose meter 102 from the cradle 103 to perform blood glucose measurement and insulin administration on the patient (Step S2707).
After completing the measurement and prescription, the user mounts the blood glucose meter 102 on the cradle 103. As a result, as is described above, the negotiation between the blood glucose meter 102 and the measurement data managing device 104 is established (Steps S2708 and S2709).
After the negotiation is established, the measurement data managing device 104 requests the blood glucose meter 102 to transfer the patient's measurement data 117 (Step S2710). Upon receiving the request, the blood glucose meter 102 creates the patient's measurement data 117 based on the internal patient table 112 and transfers the created patient's measurement data 117 to the measurement data managing device 104 (Step S2711).
The measurement data managing device 104 records the content of the received patient's measurement data 117 in the patient history table 1006 (Step S2713). Further, the content recorded in the patient history table 1006 is compared with the content previously recorded in the patient information table 1004 (Step S2714). The comparison is performed for verifying whether or not the blood glucose meter 102 has performed the operation according to the instruction.
For example, if the instruction is "blood glucose measurement only", whether or not the blood glucose measurement has been performed will be confirmed.
Similarly, if the instruction is "blood glucose measurement and insulin administration", whether or not the blood glucose measurement and the insulin administration have been performed will be confirmed.
As a result of the comparison, if there is unconformity between the instruction and the execution result even for one patient, the measurement data managing device 104 will issue a warning on the display unit 907 thereof (Step S2715).

Description will be continued below with reference to FIG. 28.
After the warning, the measurement data managing device 104 requires the operator to perform a predetermined operation (Step S2816). The operation is performed for the operator to determine whether or not the data for performing blood glucose measurement and/or insulin administration on the patient whose blood glucose measurement and/or insulin administration is unperformed (i.e., is forgotten to be performed) shall be transferred again. If the operator operates to require transferring the data again, the measurement data managing device 104 will create the data (Step S2817) and transfer the patient data 114 and the like to the blood glucose meter 102 again (Step S2818). Further, similar to that mentioned above, the predetermined data is also written in the patient information table 1004 (Step S2819).

Thereafter, the same operation as described above will continue.
The user picks up the blood glucose meter 102 from the cradle 103 to perform the blood glucose measurement and the insulin administration on the patient (Step S2820).
After completing the measurement and the prescription, the user mounts the blood glucose meter 102 on the cradle 103. As a result, the negotiation between the blood glucose meter 102 and the measurement data managing device 104 is established (Steps S2821 and S2822).
After the negotiation is established, the measurement data managing device 104 requests the blood glucose meter 102 to transfer the patient's measurement data 117 (Step S2823). Upon receiving the request, the blood glucose meter 102 creates the patient's measurement data 117 based on the internal patient table 112 and transfers the created patient's measurement data 117 to the measurement data managing device 104 (Step S2824).

The measurement data managing device 104 records the content of the received patient's measurement data 117 in the patient history table 1006 (Step S2825). Further, the content recorded in the patient history table 1006 is compared with the content previously recorded in the patient information table 1004 (Step S2826).
As a result of the comparison, if there is no unconformity between instruction and execution result for all patients, the measurement data managing device 104 will display "OK" on the display unit 907 thereof (Step S2827).

On the other hand, after the patient's measurement data 117 has been transferred to the measurement data managing device 104 (Step S2824), the blood glucose meter 102 deletes the internal patient table 112 (Step S2828). By performing this operation, it is made sure that the blood glucose meter 102 always performs blood glucose measurement or insulin administration with the newest data only.

The operation flow described above can be briefed as below.
(1) First, the patient data 114 and the like is transferred from the measurement data managing device 104 to the blood glucose meter 102.
(2) The blood glucose measurement, the insulin prescription and the like are performed.
(3) After completing the measurement and prescription, the blood glucose meter 102 is mounted on the cradle 103.
(4) As a result, the recorded patient history table 1006, which has the content of the received patient's measurement data 117 recorded therein, is compared with the patient information table 1004, and if there is any discrepancy, a warning will be displayed on the display unit 907 of the measurement data managing device 104.
(5) The patient data 114 for the incomplete patient will be transferred to the blood glucose meter 102 again.
(6) The blood glucose measurement, the insulin prescription and the like are performed.
(7) After completing the measurement and the prescription, the blood glucose meter 102 is mounted on the cradle 103.
(8) As a result, the recorded patient history table 1006, which has the content of the received patient's measurement data 117 recorded therein, is compared with the patient information table 1004, and if there is no discrepancy for all patients, the processing will be terminated.

Only data of the patients who are to be subjected to one round are stored in the blood glucose meter 102.
If there are some of the patients whose blood glucose measurement and/or insulin administration is forgotten to be performed, the measurement data managing device 104 will verify the received patient measurement data to identify the patient(s) whose blood glucose measurement and/or insulin administration has not been performed. Further, the data for performing necessary operation on the identified patient(s) is created again. In other words, "reround" is performed.
The design concept "performing only one round" of the blood glucose meter 102 is based on a concept that never allowing any margin for the error in blood glucose measurement and insulin administration.

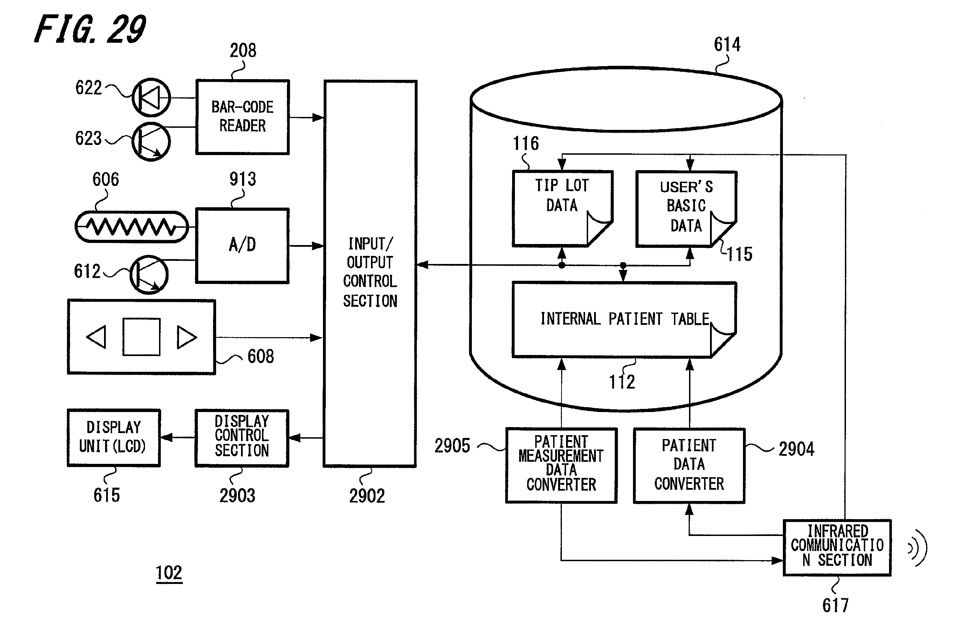

FIG. 29 is a functional block diagram of the blood glucose meter 102.
The data generated by the bar-code reader 208, the data generated by the thermistor 606, the data generated by the phototransistor 612 (a portion of the optical measuring section, also called a "blood glucose sensor"), and the data generated by the operating section 608 are inputted to an input/output control section 2902.
Further, the input/output control section 2902 performs data input/output between itself and the user's basic data 115, the tip lot data 116 and the internal patient table 112 inside the nonvolatile storage 614.
The content to be shown to the user is created by the input/output control section 2902 and displayed on the display unit 615 through a display control section 2903.
The internal patient table 112 is created by converting the patient data 114 received from the infrared communication section 617 with a patient data converter 2904. Further, the internal patient table 112 is converted into the patient's measurement data 117 by a patient measurement data converter 2905, and the patient's measurement data 117 is transmitted to the measurement data managing device 104 through the infrared communication section 617.

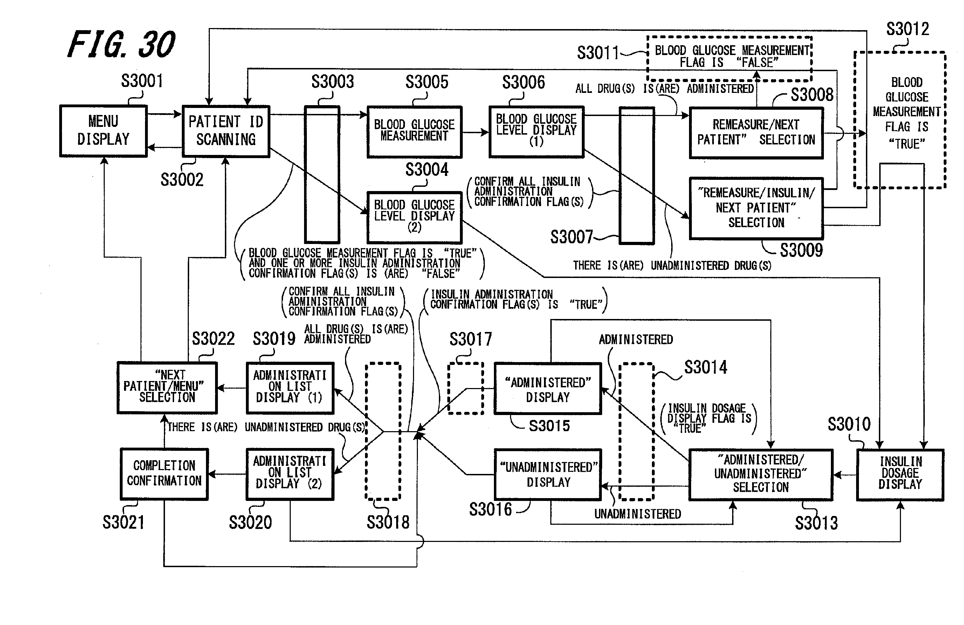

FIG. 30 is a state transition diagram of the blood glucose meter 102.
In the case where there are a plurality of insulin prescriptions to be administered, there will be a plurality of insulin administration confirmation flags set respectively for the plurality of insulin prescriptions.
First, when power source is turned on, the blood glucose meter 102 will be brought into a menu display state S3001. By performing a predetermined operation from the menu display state S3001, the blood glucose meter 102 is brought into a patient ID scanning state S3002 ready for reading the patient ID with the bar-code reader 208.
In the patient ID scanning state S3002, when the patient ID is read by the bar-code reader 208, the input/output control section 2902 will check the blood glucose measurement flag and the insulin administration confirmation flag(s) of the record of the patient ID in the internal patient table 112 (branch point S3003).
At the time of branch point S3003, in the case where the blood glucose measurement flag of the patient is "true" and one or more insulin administration confirmation flag(s) of the patient is (are) "false", the processing of the blood glucose meter 102 will proceed to a blood glucose level display (2) state S3004. If not in such a case, in other words, if in the case where the blood glucose measurement flag is "false" or where all insulin administration confirmation flag(s) is(are) "true", the processing of the blood glucose meter 102 will proceed to a blood glucose measurement state S3005.

At the time of the blood glucose measurement state S3005, after the blood glucose measurement has been performed, the processing of the blood glucose meter 102 will proceed to a blood glucose level display (1) state S3006.
Next, from the time of the blood glucose level display (1) state S3006, the input/output control section 2902 confirms all insulin administration confirmation flag(s) associated with the patient (branch point S3007). In other words, from the blood glucose level display (1) state S3006, the processing of the blood glucose meter 102 will proceed to the next state according to the state of all insulin administration confirmation flag(s) associated with the patient (branch point S3007).
At the time of the branch point S3007, if all insulin administration confirmation flag(s) is(are) "true", the processing of will proceed to a "REMEASURE/NEXT PATIENT" selection state S3008. At the time of the branch point S3007, one or more insulin administration confirmation flag(s) is(are) "false", the processing will proceed to a "REMEASURE/INSULIN/NEXT PATIENT" selection state S3009.

"one or more insulin administration confirmation flag(s) being 'false'" at the time of the branch point S3007 means that there is(are) unadministered drug(s) for the patient. In contrast, "all insulin administration confirmation flag(s) being "true'" means that all drug(s) for the patient has been administered, and there is no unadministered drug for the patient. If there is unadministered drug(s) at the time of branch point S3007, the processing can proceed to an insulin dosage display state S3010 depending on the subsequent selection performed by the operator; while if there is no unadministered, the processing can not proceed to the insulin dosage display state S3010.

In the "REMEASURE/NEXT PATIENT" selection state S3008, a character string "REMEASURE" and a character string "NEXT PATIENT" are displayed on the display unit 615 as the selection items. The operator operates the operating section 608 to select either item.
At the time of "REMEASURE/NEXT PATIENT" selection state S3008, if the operator selects the "REMEASURE", the blood glucose measurement flag will be set to "false" at Step S3011, and processing will be returned to the patient ID scanning state S3002.
At the time of "REMEASURE/NEXT PATIENT" selection state S3008, if the operator selects the "NEXT PATIENT", the blood glucose measurement flag will be set to "true" at Step S3012, and processing will be returned to the patient ID scanning state S3002.

In the "REMEASURE/INSULIN/NEXT PATIENT" selection state S3009, a character string "REMEASURE", a character string "NEXT PATIENT", and a character string "INSULIN" are displayed on the display unit 615 as the selection items. The operator operates the operating section 608 to select either item.
At the time of "REMEASURE/INSULIN/NEXT PATIENT" selection state S3009, if the operator selects the "REMEASURE", the blood glucose measurement flag will be set to "false" at Step S3011, and processing will returned to the patient ID scanning state S3002.
At the time of "REMEASURE/INSULIN/NEXT PATIENT" selection state S3009, if the operator selects the "NEXT PATIENT", the blood glucose measurement flag will be set to "true" at Step S3012, and processing will be returned to the patient ID scanning state S3002.
At the time of "REMEASURE/INSULIN/NEXT PATIENT" selection state S3009, if the operator selects the "INSULIN", the blood glucose measurement flag will be set to "true" at Step S3012, and processing will proceed to the insulin dosage display state S3010.

The processing proceeds to the insulin dosage display state S3010 either at the time when the operator selects the "INSULIN" at the time of "REMEASURE/INSULIN/NEXT PATIENT" selection state S3009, or after the blood glucose level display (2) state S3004.
In the insulin dosage display state S3010, kind and dosage of the insulin to be administered to the patient are displayed on the display unit 615.
In the insulin dosage display state S3010, only the Enter key 206 is in an operable state. Further, when the Enter key 206 is pressed, the processing of the blood glucose meter 102 will proceed to an "ADMINISTERED/UNADMINISTERED" selection state S3013.

In the "ADMINISTERED/UNADMINISTERED" selection state S3013, a character string "ADMINISTERED" and a character string "UNADMINISTERED" are displayed on the display unit 615 as the selection items. The operator operates the operating section 608 to select either item.
At the time of "ADMINISTERED/UNADMINISTERED" selection state S3013, if the operator selects the "ADMINISTERED", the insulin dosage display flag will be set to "true" at Step S3014, and processing will proceed to an "ADMINISTERED" display state S3015.
At the time of "ADMINISTERED/UNADMINISTERED" selection state S3013, if the operator selects the "UNADMINISTERED", the insulin dosage display flag will be set to "true" at Step S3014, and processing will proceed to an "UNADMINISTERED" display state S3016.

In the "ADMINISTERED" display state S3015, in addition to a display-dedicated character string "ADMINISTERED", a character string "OK" and a character string "RETURN" are displayed on the display unit 615 as the selection items. The operator operates the operating section 608 to select either of the "OK" or "RETURN".
At the time of "ADMINISTERED" display state S3015, if the operator selects the "OK", the insulin administration confirmation flag(s) associated with the drug(s) displayed in the insulin dosage display state S3010 will be set to "true" in Step S3017, and the processing will proceed a branch point S3018.
At the time of the "ADMINISTERED" display state S3015, when the operator selects "RETURN", the processing will be returned to the "ADMINISTERED/UNADMINISTERED" selection state S3013. Such selection is provided for preventing incorrect operation.

In the "UNADMINISTERED" display state S3016, in addition to the display-dedicated character string "UNADMINISTERED", a character string "OK" and a character string "RETURN" are displayed on the display unit 615 as the selection items. The operator operates the operating section 608 to select either of the "OK" or "RETURN".
At the time of the "UNADMINISTERED" display state S3016, when the operator selects "OK", the processing will proceed to the branch point S3018.
In other words, unlike the "ADMINISTERED" display state S3015, in the "UNADMINISTERED" display state S3016, the insulin administration confirmation flag(s) associated with the drug(s) displayed in the insulin dosage display state S3010 will not be set to "true".
At the time of the "UNADMINISTERED" display state S3016, when the operator selects "RETURN", the processing will be returned to the "ADMINISTERED/UNADMINISTERED" selection state S3013. Such selection is provided for preventing incorrect operation.

At the branch point S3018, the state of all insulin administration confirmation flag(s) associated with the patient is conformed. If all insulin administration confirmation flag(s) of the patient is(are) "true", i.e., if all drug(s) is(are) administered, the processing will proceed to an administration list display (1) state S3019.
If one or more of the insulin administration confirmation flag(s) of the patient is(are) "false", i.e., if there is any unadministered drug, the processing will proceed to an administration list display (2) state S3020.

In the administration list display (1) state S3019, in addition to the list of the drug(s) administered to the patient, a character string "COMPLETION" is displayed on the display unit 615 as the only selection item. When the operator presses the Enter key 206 after confirming the content of the list of the drug(s) administered to the patient, the processing will proceed to a "NEXT PATIENT/MENU" selection state S3022.
Incidentally, in the administration list display (1) state S3019, administration of the drug(s) necessary for the patient is(are) all completed.

In the administration list display (2) state S3020, in addition to the list of the drug(s) administered to the patient, a character string "COMPLETION" and a character string "RETURN" are displayed on the display unit 615 as the selection items. After confirming the content of the list of the drug(s) administered to the patient, the operator operates the operating section 608 to select either of the "COMPLETION" or the "RETURN".
At the time of the administration list display (2) state S3020, when the operator selects "COMPLETION", the processing will proceed to a completion confirming state S3021.
At the time of the administration list display (2) state S3020, when the operator selects "RETURN", the processing will be returned to the insulin dosage display state S3010.
Incidentally, in the administration list display (2) state S3020, administration of the drug(s) necessary for the patient is not completed. Thus, when the processing is returned to the insulin dosage display state S3010, insulin dosage of the unadministered drug(s) is displayed.

In the completion confirming state S3021, in addition to a character string "THERE IS UNADMINISTERED DRUG. IS IT OK?" as a caution to the operator, a character string "RETURN" and a character string "YES" are displayed on the display unit 615 as the selection items. The operator operates the operating section 608 to select either of the "RETURN" or "YES".
At the time of the completion confirming state S3021, when the operator selects "RETURN", the processing will be returned to the branch point S3018. Further, since there is(are) unadministered drug(s), the processing will be returned to the administration list display (2) state S3020 again.
At the time of the completion confirming state S3021, when the operator selects "YES", the processing will proceed to the "NEXT PATIENT/MENU" selection state S3022.

In the "NEXT PATIENT/MENU" selection state S3022, a character string "NEXT PATIENT" and a character string "MENU" are displayed on the display unit 615 as the selection items. The operator operates the operating section 608 to select either of the "NEXT PATIENT" or "MENU".
At the time of the "NEXT PATIENT/MENU" selection state S3022, when the operator selects "NEXT PATIENT", the processing will be returned to the patient ID scanning state S3002.
At the time of the "NEXT PATIENT/MENU" selection state S3022, when the operator selects "MENU", the processing will be returned to the menu display state S3001.

In the state transition diagrams described above, there are three scenes where the input/output control section 2902 confirms the content of the flags.
One is the time when the input/output control section 2902 checks combination of the logical value of the blood glucose measurement flag and the logical value of all insulin administration confirmation flag(s) (branch point S3003) after the patient ID has been read in the patient ID scanning state S3002.
Another is the time when the input/output control section 2902 checks the logical value of all insulin administration confirmation flag(s) (branch point S3007) after the blood glucose level is displayed in the blood glucose level display (1) state S3006.
Further another is the time when the input/output control section 2902 checks the logical value of all insulin administration confirmation flag(s) (branch point S3018) after the "ADMINISTERED" display state S3015 and the "UNADMINISTERED" display state S3016.

Further, in the state transition diagrams described above, there are four scenes where the input/output control section 2902 rewrites the flags.
One is the process of setting the blood glucose measurement flag to "false" (Step S3011) in the "REMEASURE/NEXT PATIENT" selection state S3008 and the "REMEASURE/INSULIN/NEXT PATIENT" selection state S3009.
Another is the process of setting the blood glucose measurement flag to "true" (Step S3012) in the "REMEASURE/NEXT PATIENT" selection state S3008 and the "REMEASURE/INSULIN/NEXT PATIENT" selection state S3009.
Further another is the process of setting the insulin dosage display flag to "true" (Step S3014) after the "ADMINISTERED/UNADMINISTERED" selection state S3013.
Further another is the process of setting the insulin administration confirmation flag to "true" (Step S3017) when the operator selects "OK" at the time of the "ADMINISTERED" display state S3015.

In the case where blood glucose measurement is performed on a patient and then insulin administration is performed on the same patient, the processing flow will be: S3002 - S3005 - S3006 - S3009 - S3010 - S3013 - S3015 - S3019 (or S3020) - S3002.
In the case where blood glucose measurement is performed on a patient and then blood glucose measurement is performed on the next patient without performing insulin administration on the first patient, the processing flow will be: S3002 - S3005 - S3006 - S3009 - S3002.
In the case where insulin administration is performed on a patient whose blood glucose has been measured or where only insulin administration is performed from the beginning, the processing flow will be: S3002 - S3004 - S3010 - S3013 - S3015 - S3019 (or S3020) - S3002.
In the case where only blood glucose measurement is performed on a patient from the beginning or where blood glucose measurement is performed again on a patient whose insulin dosage has been displayed, the processing flow will be: S3002 - S3005 - S3006 - S3008 - S3002.

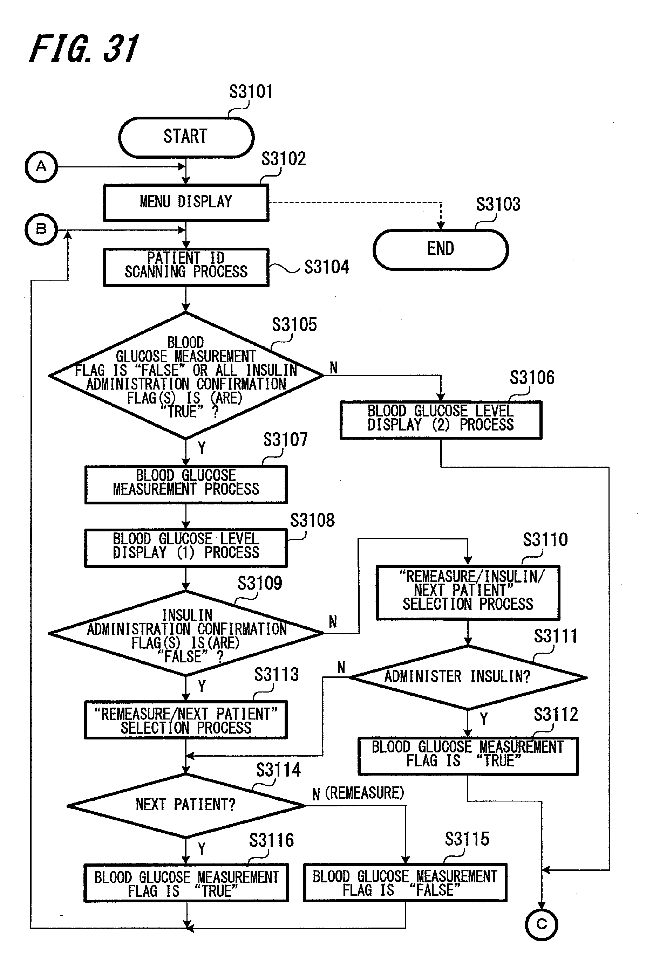

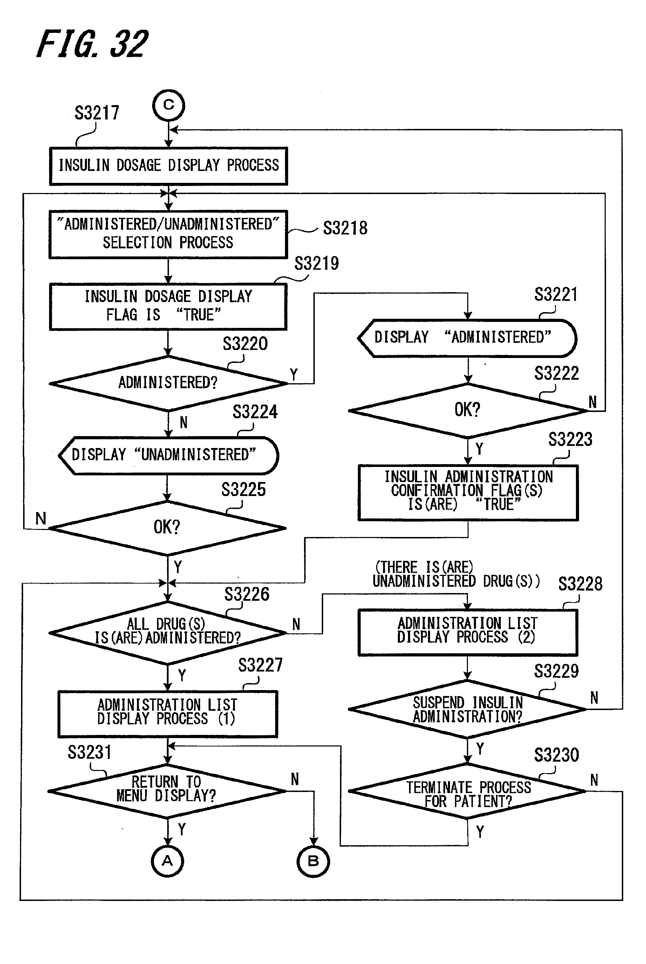

FIGS. 31 and 32 are flowcharts showing the processing flow of the blood glucose meter 102. FIGS. 31 and 32 are provided to explain the operation flow of the state transition diagram shown in FIG. 30.
When processing is started by turning on the power switch 204 (Step S3101), the blood glucose meter 102 performs menu display process first (Step S3102, corresponding to Step S3001 of FIG. 30). Here, when turning off the power switch 204, the processing will be terminated (Step S3103).
After the menu display process (Step S3102) is completed, the operator operates the blood glucose meter 102 to make it perform the patient ID scanning process (Step S3104, corresponding to Step S3002 of FIG. 30) in which the patient ID is read by the bar-code reader 208.
When the patient ID is read in the patient ID scanning process (Step S3104), the input/output control section 2902 checks the blood glucose measurement flag and all insulin administration confirmation flags in the record of the patient ID in the internal patient table 112 (Step S3105, corresponding to Step S3003 of FIG. 30).
Here, in the case where the blood glucose measurement flag is "true" and one or more insulin administration confirmation flag(s) is(are) "false" ("NO" in Step S3105), the blood glucose meter 102 will perform a blood glucose level display (2) process (Step S3106, corresponding to Step S3004 of FIG. 30). If not in such a case, in other words, in the case where the blood glucose measurement flag is "false" or where all insulin administration confirmation flag(s) is/are "true" ("YES" in Step S3105), the blood glucose meter 102 will perform a blood glucose measurement process (Step S3107, corresponding to Step S3005 of FIG. 30).

When blood glucose measurement is performed in the blood glucose measurement process (Step S3107), the blood glucose meter 102 will perform a blood glucose level display (1) process (Step S3108, corresponding to Step S3006 of FIG. 30).
When the blood glucose level is displayed in the blood glucose level display (1) process (Step S3108), the input/output control section 2902 will confirm all insulin administration confirmation flag(s) (Step S3109, corresponding to Step S3007 of FIG. 30). If all insulin administration confirmation flag(s) is(are) "true" ("YES" in Step S3109), a "REMEASURE/NEXT PATIENT" selection process will be performed (Step S3112, corresponding to the "REMEASURE/NEXT PATIENT" selection state S3008 of FIG. 30). If not in such a case, In other words, if in the case where one or more insulin administration confirmation flag(s) is(are) "false" ("NO" in Step S3109), the blood glucose meter 102 will perform a "REMEASURE/INSULIN/NEXT PATIENT" selection process (Step S3110, corresponding to the "REMEASURE/INSULIN/NEXT PATIENT" selection state S3009 of FIG. 30).

After the "REMEASURE/INSULIN/NEXT PATIENT" selection process S3110, the processing is branched depending on the operation performed by the operator.
When the operator selects the "INSULIN" ("YES" in Step S3111), the blood glucose measurement flag of the patient will be set to "true" (Step S3112), and further, the processing will proceed to an insulin dosage display process (Step S3217 in FIG. 32, corresponding to the insulin dosage display state S3010 of FIG. 30).
When the operator selects the "NEXT PATIENT" ("YES" in Step S3114), the blood glucose measurement flag of the patient will be set to "true" (Step S3116), and further, the processing will be returned to the patient ID scanning process (Step S3104).
When the operator selects the "REMEASURE" ("NO" in Step S3114), the blood glucose measurement flag of the patient will be set to "false" (Step S3115), and further, the processing will be returned to the patient ID scanning process (Step S3104).

Description will be continued below with reference to FIG. 32.
After the insulin dosage display process (Step S3217) is completed, the processing of the blood glucose meter 102 proceeds to an "ADMINISTERED/UNADMINISTERED" selection process (Step S3218, corresponding to the "ADMINISTERED/UNADMINISTERED" selection state S3013 of FIG. 30) upon receiving an operation of Enter key 206 from the operator. Thereafter, the insulin dosage display flag is set to "true" (Step S3219, corresponding to Step S3014 of FIG. 30) regardless of the result of the selection operation of the operating section 608 performed by the operator.
After Step S3219, if "ADMINISTERED" is selected ("YES" in Step S3220) as the result of the selection operation of the operating section 608 performed by the operator, a character string "ADMINISTERED" will be displayed on the display unit 615 of the blood glucose meter 102 (Step S3221, corresponding to the "ADMINISTERED" display state S3015).
After Step S3219, if "UNADMINISTERED" is selected ("NO" in Step S3220) as the result of the selection operation of the operating section 608 performed by the operator, a character string "UNADMINISTERED" will be displayed on the display unit 615 of the blood glucose meter 102 (Step S3224, corresponding to the "UNADMINISTERED" display state S3016).

After the character string "ADMINISTERED" is displayed on the display unit 615 in Step S3221, the processing of the blood glucose meter 102 will be branched depending on the result of the selection operation of the operating section 608 performed by the operator.
After Step S3221, if "OK" is selected ("YES" in Step S3222) as the result of the selection operation of the operating section 608 performed by the operator, the insulin administration confirmation flag(s) in the blood glucose meter 102 will be set to "true" (Step S3223, corresponding to Step S3017 of FIG. 30) and then the processing will proceed to a branch of Step S3226.
After Step S3221, if "RETURN" is selected ("NO" in Step S3222) as the result of the selection operation of the operating section 608 performed by the operator, the processing of the blood glucose meter 102 will be returned to the "ADMINISTERED/UNADMINISTERED" selection process (Step S3218).

Similar to Step S3221, after the character string "UNADMINISTERED" is displayed on the display unit 615 in Step S3224, the processing of the blood glucose meter 102 will be branched depending on the result of the selection operation of the operating section 608 performed by the operator.
After Step S3224, if "OK" is selected ("YES" in Step S3225) as the result of the selection operation of the operating section 608 performed by the operator, the processing of the blood glucose meter 102 will proceed to a branch of Step S3226.
After Step S3224, if "RETURN" is selected ("NO" in Step S3225) as the result of the selection operation of the operating section 608 performed by the operator, the processing of the blood glucose meter 102 will be returned to the "ADMINISTERED/UNADMINISTERED" selection process (Step S3218).

In Step S3226, the blood glucose meter 102 confirms all insulin administration confirmation flag(s) of the patient currently subjected to treatment.
If all insulin administration confirmation flag(s) is/are "true", i.e., if all drug(s) is(are) administered ("YES" in Step S3226), the processing of the blood glucose meter 102 will proceed to an administration list display process (1) (Step S3227, corresponding to the administration list display (1) state S3019 of FIG. 30).
If one or more insulin administration confirmation flag(s) is(are) "false", i.e., if there is(are) unadministered drug(s) ("NO" in Step S3226), the processing of the blood glucose meter 102 will proceed to an administration list display process (2) (Step S3228, corresponding to the administration list display (2) state S3020 of FIG. 30).

After the administration list display process (2) of Step S3228, the processing of the blood glucose meter 102 will be branched depending on the result of the selection operation of the operating section 608 performed by the operator.
After Step S3228, if "YES" is selected ("YES" in Step S3229, corresponding to the administration list display (2) state S3020 of FIG. 30) as the result of the selection operation of the operating section 608 performed by the operator, the blood glucose meter 102 will judge that the operator has suspended the remaining insulin administration process for the patient, and therefore the processing will proceed to a branch processing (Step S3230, corresponding to the completion confirming state S3021 of FIG. 30) for further confirmation.
After Step S3228, if "RETURN" is selected ("NO" in Step S3229, corresponding to the administration list display (2) state S3020 of FIG. 30) as the result of the selection operation of the operating section 608 performed by the operator, the blood glucose meter 102 will judge that the operator decides to continue the remaining insulin administration process for the patient, and therefore the processing will proceed to the insulin dosage display process (Step S3217).

In the branch processing for confirmation in Step S3230, the processing of the blood glucose meter 102 is branched depending on the result of the selection operation of the operating section 608 performed by the operator.
If "YES" is selected ("YES" in Step S3230, corresponding to the completion confirming state S3021 of FIG. 30) as the result of the selection operation of the operating section 608 performed by the operator, the blood glucose meter 102 will judge that the operator has confirmed his(or her) decision to suspend the remaining insulin administration process for the patient, and therefore the processing will proceed to a branch processing for querying whether or not the menu display state shall be returned (Step S3231, corresponding to the "NEXT PATIENT/MENU" selection state S3022 of FIG. 30).
If "RETURN" is selected ("NO" in Step S3230, corresponding to the completion confirming state S3021 of FIG. 30) as the result of the selection operation of the operating section 608 performed by the operator, the blood glucose meter 102 will judge that the operator want to restart the remaining insulin administration process for the patient, and the processing will proceed to a branch processing of Step S3226. As a result, since there is(are) unadministered drug(s) ("YES" in Step S3226), the processing proceeds to the administration list display process (2) of Step S3228 again.

In the branch processing for querying whether or not the menu display state shall be returned, the processing of the blood glucose meter 102 is branched depending on the result of the selection operation of the operating section 608 performed by the operator.
If "MENU" is selected ("YES" in Step S3231, corresponding to the "NEXT PATIENT/MENU" selection state S3022 of FIG. 30) as the result of the selection operation of the operating section 608 performed by the operator, the processing of the blood glucose meter 102 will be returned to the menu display process (Step S3102 of FIG. 31).
If "NEXT PATIENT" is selected ("NO" in Step S3231, corresponding to the "NEXT PATIENT/MENU" selection state S3022 of FIG. 30) as the result of the selection operation of the operating section 608 performed by the operator, the processing of the blood glucose meter 102 will be returned to the patient ID scanning process (Step S3102 of FIG. 31).

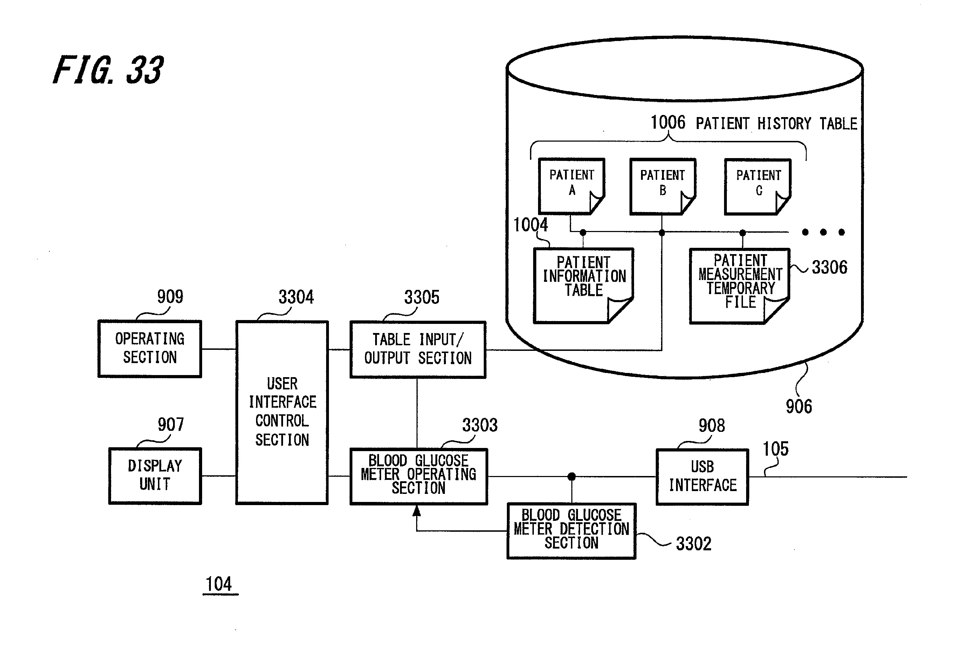

FIG. 33 is a functional block diagram of the measurement data managing device 104.
Note that, in the present embodiment, only the function of the measurement data managing device 104 associated with the comparison between the patient information table 1004 and the patient history table 1006 will be described, although the measurement data managing device 104 also has various other functions not included in the disclosure of the present embodiment.

A blood glucose meter detecting section 3302 constantly monitors whether or not the blood glucose meter 102 is mounted on the cradle 103 through the USB interface 908. When the blood glucose meter 102 is mounted on the cradle 103 or when the blood glucose meter 102 is removed from the cradle 103, the blood glucose meter detecting section 3302 reports such fact to a blood glucose meter operating section 3303.

The blood glucose meter operating section 3303 mainly performs the following two operations.
One is essential data communication operation performed immediately after the blood glucose meter detecting section 3302 reports that the blood glucose meter 102 is mounted on the cradle 103. The essential data communication operation is performed basically without making any inquiry to the user.
The other is optional data communication operation for transmitting necessary data to the blood glucose meter 102 for it to perform round. The optional data communication operation is performed in response to the operation performed by the user.
In other words, basically the operation for the measurement data managing device 104 to collect the patient's measurement data 117 and the like from the blood glucose meter 102 is performed automatically as soon as the blood glucose meter 102 is mounted on the cradle 103.
On the other hand, the operation for the measurement data managing device 104 to transmit the patient data 114 and the like to the blood glucose meter 102 has to be performed in response to (manual) operation performed by the user.

The operating section 909 is configured by a keyboard, a mouse and/or the like. The display unit 907 is an LCD or the like. The operating section 909 and the display unit 907 are connected to a user interface control section 3304.
The user interface control section 3304 makes the display unit 907 to display a predetermined operation screen. Further, when receiving user's operation from the operating section 909, the user interface control section 3304 changes the operation screen displayed on the display unit 907, sends a predetermined instruction to the blood glucose meter detecting section 3302, and inputs/outputs necessary data to/from a table input/output section 3305.

The table input/output section 3305 is an interface for performing data input/output operation between many tables stored in the nonvolatile storage 906 of the measurement data managing device 104 (such as the patient history table 1006, the patient information table 1004, a patient measurement temporary file 3306 and the like), the user interface control section 3304 and the blood glucose meter operating section 3303. To be specific, the table input/output section 3305 is a database manager called "middleware".

The measurement data managing device 104 is necessary for storing large amounts of data. Particularly, since the patient's measurement data 117 is downloaded from the blood glucose meter 102 and stored in the nonvolatile storage 906 every time when round is completed, volumes of data will increase day by day. To cope with the large amounts of data and achieve quick data input/output function, it is preferred to employ the middleware. Further, by employing the middleware, efficiency of program construction can be improved.
Note that, although only the patient history table 1006 and the patient information table 1004 are shown in FIG. 33, there are actually more tables. In FIG. 26, the tables unnecessary for describing the present embodiment are omitted.

The patient history table 1006 is created for each patient. For this reason, each table name of the patient history table 1006 includes a patient ID. Such arrangement is made for the sake of efficiency when storing data. Incidentally, the patient history table 1006 may also be configured as a single table having a patient ID field.

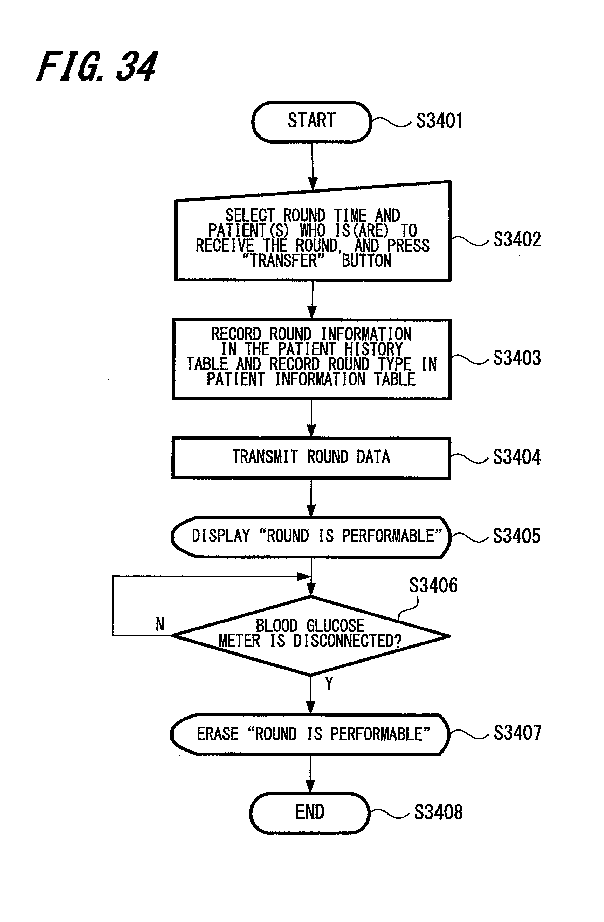

FIG. 34 is a flowchart showing the processing flow when patient data 114 or the like is transferred from the measurement data managing device 104 to the blood glucose meter 102.
When processing is started (Step S3401), the measurement data managing device 104 is in a state of waiting for operation from the user. The user selects round time and patient(s) who is(are) to receive the round, and presses a "TRANSFER" button displayed on the display unit 907 (Step S3402). As a result, the operation performed by the user is inputted from the operating section 909 to the user interface control section 3304. The user interface control section 3304 controls the table input/output section 3305 to record round information for each patient designated by the user in the patient history table 1006 and record information on whether or not blood glucose measurement has been performed and whether or not insulin administration has been performed (round type) in a "ROUND TYPE IN PROCESS" field (Step S3403).

Next, based on the content recorded in the patient history table 1006 and the patient information table 1004, the user interface control section 3304 creates the patient data 114 through the table input/output section 3305. Further, the user interface control section 3304 controls the table input/output section 3305 and the blood glucose meter operating section 3303 to transmit the patient data 114, together with the user's basic data 115 and the tip lot data 116, to the blood glucose meter 102 through the USB interface 908 (Step S3404). After transmission is completed, the user interface control section 3304 makes the display unit 907 to display a character string "ROUND IS PERFORMABLE" (Step S3405).

Next, the blood glucose meter operating section 3303 confirms whether or not the blood glucose meter detecting section 3302 has lost sight of the blood glucose meter 102 (Step S3406). If the blood glucose meter 102 is disconnected ("YES" in Step S3406), the blood glucose meter operating section 3303 will report such fact to the user interface control section 3304. Upon receiving such a report, the user interface control section 3304 will erase the character string "ROUND IS PERFORMABLE" displayed on the display unit 907 (Step S3407), and the processing will be terminated (Step S3408).

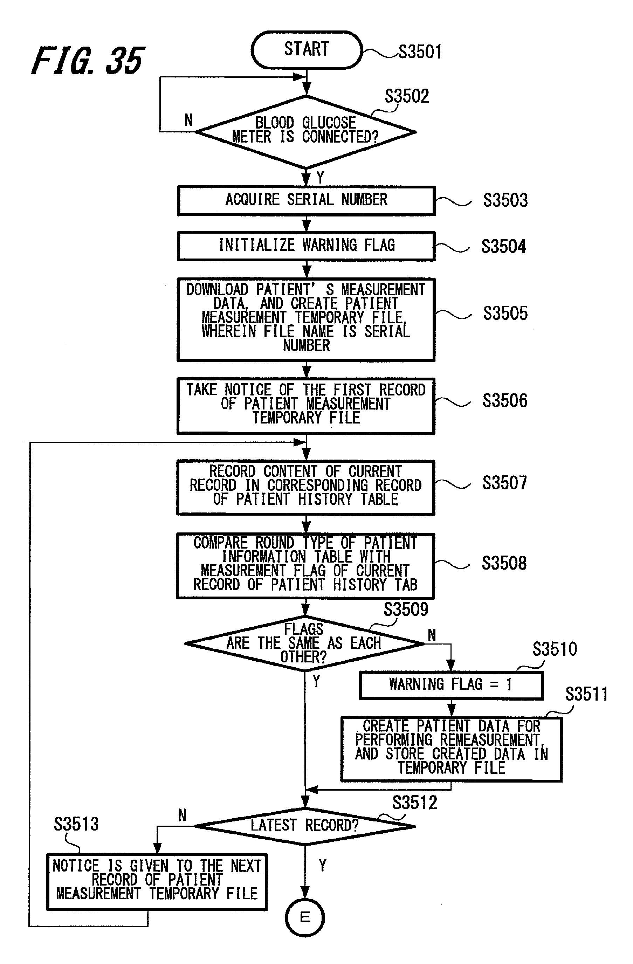

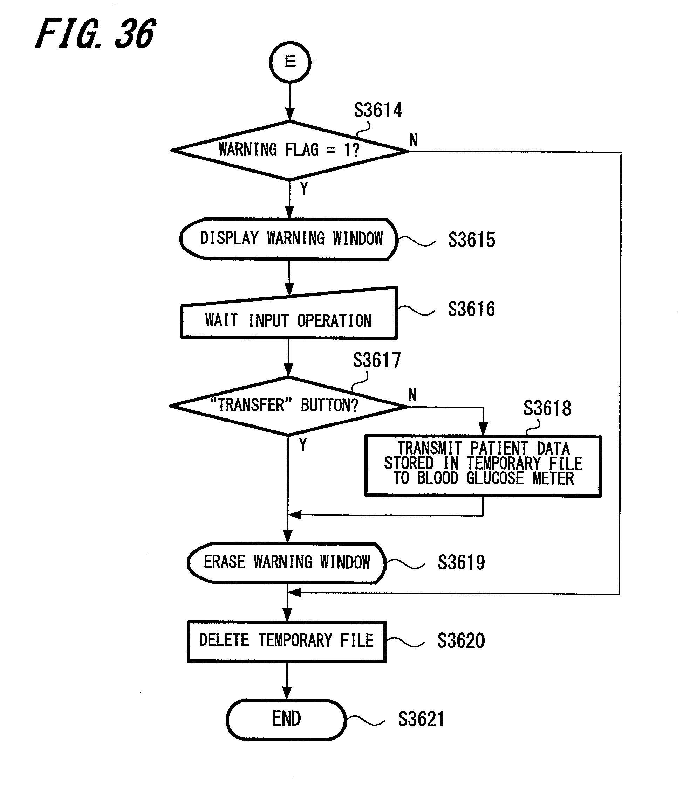

FIGS. 35 and 36 are flowcharts showing the processing flow when the measurement data managing device 104 receives the patient's measurement data 117 or the like from the blood glucose meter 102.
When the processing is started (Step S3501), the blood glucose meter operating section 3303 confirms whether or not the blood glucose meter detecting section 3302 has detected the connection of the blood glucose meter 102 (Step S3502). When the blood glucose meter 102 is connected ("YES" in Step S3502), first the blood glucose meter operating section 3303 acquires the serial number of the blood glucose meter 102 (Step S3503). Next, the user interface control section 3304 sets an internal warning flag (not shown in the drawings) to "false" (i.e., initialization) (Step S3504).

Next, the blood glucose meter operating section 3303 downloads the patient's measurement data 117 from the blood glucose meter 102, and creates the patient measurement temporary file 3306 through the table input/output section 3305 (Step S3505), wherein the file name of the patient measurement temporary file 3306 is the previously acquired serial number. In response, the user interface control section 3304 take notice of the first record of the patient measurement temporary file 3306 (Step S3506).

The following is a loop processing.
The user interface control section 3304 reads the currently noticed record of the patient measurement temporary file 3306, and records the content of the current record in the corresponding record of the corresponding patient history table 1006. Next, the round type, i.e., the information on whether or not blood glucose measurement has been performed and whether or not insulin administration has been performed, recorded in the corresponding record of the patient information table 1004 is compared with the previously recorded content of the flag recorded in an "INSULIN ADMINISTRATION RESULT" field in the current record of the patient history table 1006.
As a result of the comparison, if there is any discrepancy in content ("NO" in Step S3509), the user interface control section 3304 will set the warning flag to "true" (Step S3510). Further, the patient data 114 for performing remeasurement is created based on the data currently recorded in the patient history table 1006, and the created patient data 114 is stored in a temporary file (not shown in the drawings) (Step S3511).
As a result of the comparison, if there is no difference in content ("YES" in Step S3509), no operation will be done.
Further, the user interface control section 3304 confirms whether or not the currently noticed record of the patient measurement temporary file 3306 is the latest record (Step S3512). If the currently noticed record of the patient measurement temporary file 3306 is not the last record ("NO" in Step S3512), notice will be taken to the processing will be terminated (Step S3507).

Description will be continued below with reference to FIG. 36.
In Step S3512, if the currently noticed record is the last record ("YES" in Step S3512), the user interface control section 3304 will confirm the warning flag (Step S3614). If the warning flag is "true" ("YES" in Step S3614), a warning message will be displayed (Step S3615). Further, input operation from the user will be waited (Step S3616).
If the user presses the "TRANSFER" button ("YES" in Step S3617), the warning message will be erased without do anything. However, if the pressed button is not the "TRANSFER" button ("NO" in Step S3617), the user interface control section 3304 will control the table input/output section 3305 and the blood glucose meter operating section 3303 to transmit the patient data 114 previously created and stored in the temporary file to the blood glucose meter 102 (Step S3618).
No matter what button is selected, the user interface control section 3304 will finally delete the temporary file (Step S3620), and the processing will be terminated (Step S3621).

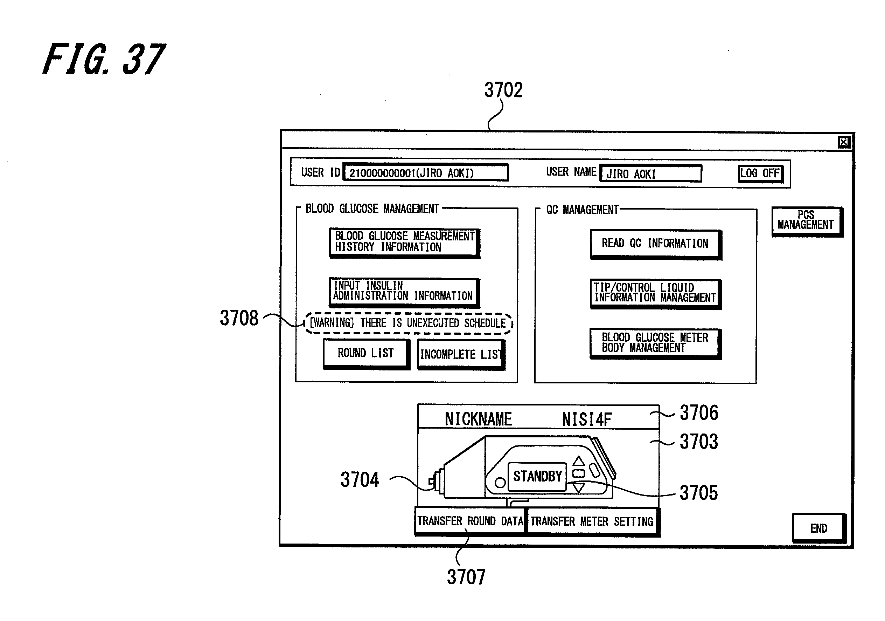

FIG. 37 is a view showing a main menu window displayed on the display unit 907 of the measurement data managing device 104. FIG. 37 shows an example of the warning display.
A blood glucose meter state display column 3703 is provided in the central lower portion of a main menu window 3702. An illustration 3704 of the blood glucose meter 102 is displayed on the blood glucose meter state display column 3703. A portion of the illustration 3704 corresponding to the LCD is a connection state display column 3705.
A nickname formed by any character string given to the blood glucose meter 102 is displayed in a nickname display column 3706.
A warning message 3708 will be displayed when the measurement data managing device 104 judges that there is any patient whose blood glucose is forgotten to be measured and/or whose insulin is forgotten to be administered. A message "[WARNING] THERE IS UNEXECUTED SCHEDULE" is displayed in FIG. 37. Whether or not the warning message shall be displayed is judged by confirming whether or not the "ROUND TYPE IN PROCESS" field of the patient information table 1004 and the "INSULIN ADMINISTRATION RESULT" field of the patient history table 1006 are coincidence/noncoincidence with each other.

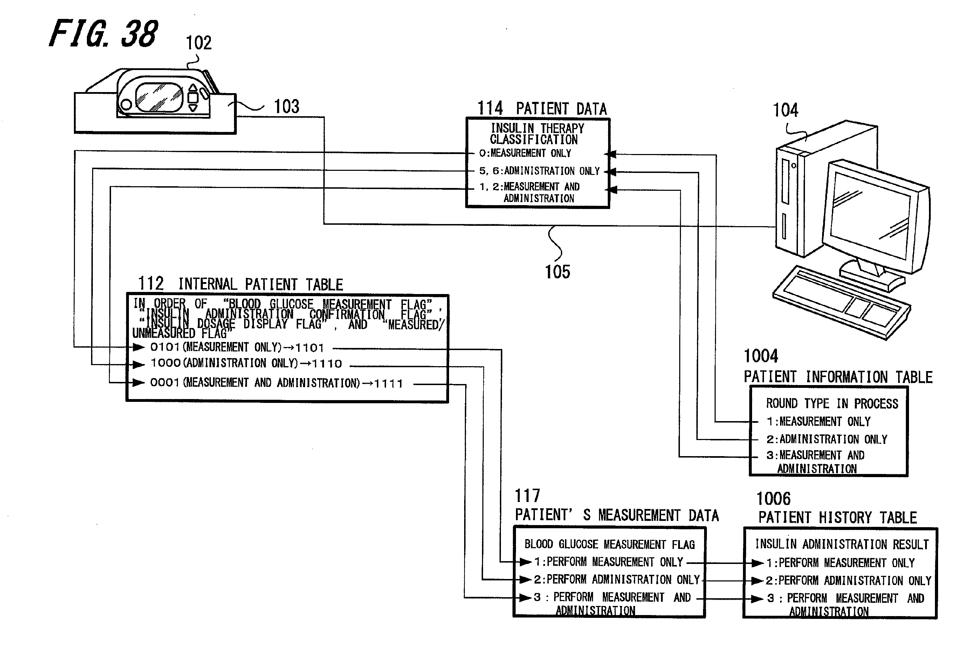

FIG. 38 is a view schematically showing change of the flags contained in the data and tables existing between the measurement data managing device 104 and the blood glucose meter 102.
The "ROUND TYPE IN PROCESS" field is arranged in the patient information table 1004 stored in the nonvolatile storage of the measurement data managing device 104. The "ROUND TYPE IN PROCESS" field has the following contents recorded therein:
· "1", when only blood glucose measurement is performed.
· "2", when only insulin administration is performed.
· "3", when both the blood glucose measurement and the insulin administration are performed.

These contents indicate the instruction for the blood glucose meter 102 to perform.

The patient data 114 is created based on the contents recorded in the patient information table 1004 and the patient history table 1006. The patient data 114 has the "INSULIN THERAPY CLASSIFICATION" field. The "INSULIN THERAPY CLASSIFICATION" field has the following contents recorded therein:
· "0", in the case where only "blood glucose measurement" is performed.
· "1", in the case where "blood glucose measurement" and "insulin administration using sliding scale" are performed.
· "2", in the case where "blood glucose measurement" and "fixed-dose insulin administration" are performed.
· "5", in the case where only "insulin administration using sliding scale" is performed.
· "6", in the case where only "fixed-dose insulin administration" is performed.

After being read to the blood glucose meter 102, the patient data 114 is recorded in the internal patient table 112. Here, the value of the "INSULIN THERAPY CLASSIFICATION" field of the patient data 114 is converted into the following four flags:
· Blood glucose measurement flag;
· Insulin administration confirmation flag:
· Insulin dosage display flag; and
· Measured/unmeasured flag.
Among these flags, the blood glucose measurement flag, the insulin administration confirmation flag and the insulin dosage display flag are the value of the "MEASUREMENT/DISPLAY/ADMINISTRATION FLAG" field, and the measured/unmeasured flag is the value of the "MEASURED/UNMEASURED FLAG" field.

In the case where only blood glucose measurement is performed, the blood glucose measurement flag will be set to "false", and, since insulin administration is not performed, the insulin administration confirmation flag(s) will be set to "true".
Since the insulin dosage display flag is set to "true" only by performing the insulin dosage display process, initial value thereof must be "false".
Since the blood glucose measurement is to be performed, the measured/unmeasured flag is set to "true".
In other words, these flags are set to "0101" in the same order thereof.
Incidentally, since the insulin administration confirmation flag(s) is(are) data accompanied with the data of the drug(s) to be administered, in the case where only blood glucose measurement is performed, dummy drug administration information is applied to the patient so that the insulin administration confirmation flag(s) can be assigned.

In the case where only insulin administration is performed, the blood glucose measurement flag will be set to "true", and, since insulin administration needs to be performed, all insulin administration confirmation flag(s) for the drug(s) to be administered to the patient is(are) set to "false".
As is described above, the initial value of each of the insulin dosage display flag(s) is "false".
Since the blood glucose measurement is not to be performed, the measured/unmeasured flag is set to "false".
In other words, these flags are set to "1000" in the same order thereof.

In the case where both the blood glucose measurement and the insulin administration are performed, the blood glucose measurement flag will be set to "false", the insulin administration confirmation flag will be set to "false", the insulin dosage display flag will be set to "false", and the measured/unmeasured flag will be set to "true". Thus, these flags are set to "0001" in the same order thereof.

In the case where only blood glucose measurement is performed, since the blood glucose measurement flag is set to "true" after the blood glucose measurement is completed, the setting of the flags in changed from "0101" to "1101".

In the case where only insulin administration is performed, since the insulin administration confirmation flag(s) and the insulin dosage display flag are respectively set to "true" after the insulin administration is completed, the setting of the flags will be changed from "1000" to "1110".

In the case where both the blood glucose measurement and the insulin administration are performed, since the blood glucose measurement flag, the insulin administration confirmation flag(s) and the insulin dosage display flag are all set to "true" after both the blood glucose measurement and the insulin administration are completed, the setting of the flags will be changed from "0001" to "1111".

The values of these flags of the internal patient table 112 are converted to the value of the "BLOOD GLUCOSE MEASUREMENT FLAG" field.
- In the case where the flags are "1101", the values of these flags are converted to "1" which means that only blood glucose measurement is completed.
- In the case where the flags are "1110", the values of these flags are converted to "2" which means that only insulin administration is completed.
- In the case where the flags are "1111", the values of these flags are converted to "3" which means that both the blood glucose measurement and the insulin administration are completed.
The converted values are recorded in the "INSULIN ADMINISTRATION RESULT" field of the patient history table 1006 as they are.

The value of the "ROUND TYPE IN PROCESS" field of the patient information table 1004 and the value of the "INSULIN ADMINISTRATION RESULT" field of the patient history table 1006 are compared with each other. If both values are equal to each other, it will be judged that the instructed operation has been performed, and if both values are not equal to each other, it will be judged that the instructed operation has not been performed.

If the value of the respective flags stays unchanged from "0101", "1000" or "0001", it indicates that both the blood glucose measurement and the insulin administration have not been performed. In such a case, "0" is recorded in the "BLOOD GLUCOSE MEASUREMENT FLAG" field of the patient's measurement data 117.
Further, when instruction is "blood glucose measurement and insulin administration", there is a possible case where only blood glucose measurement has been performed, and insulin administration is forgotten. In such a case, the value of the flags will be changed from "0001" to "1001", which means only blood glucose measurement has been performed, but insulin administration confirmation is forgotten, or to "1101", which means blood glucose measurement and insulin administration confirmation have been performed, but insulin dosage display has non been performed. These two values of the flags correspond to "1", which means "performing blood glucose measurement only", of the "BLOOD GLUCOSE MEASUREMENT FLAG" field of the patient's measurement data 117.

When the value of the "ROUND TYPE IN PROCESS" field of the patient information table 1004 is "1" which means "only performing blood glucose measurement", in the case where the value of the "INSULIN ADMINISTRATION RESULT" field of the patient history table 1006 is "0", it will be judged that blood glucose measurement is forgotten to be performed on the patient.
When the value of the "ROUND TYPE IN PROCESS" field of the patient information table 1004 is "2" which means "only performing insulin administration", in the case where the value of the "INSULIN ADMINISTRATION RESULT" field of the patient history table 1006 is "0", it will be judged that insulin administration is forgotten to be performed on the patient.
When the value of the "ROUND TYPE IN PROCESS" field of the patient information table 1004 is "3" which means "performing blood glucose measurement and insulin administration", in the case where the value of the "INSULIN ADMINISTRATION RESULT" field of the patient history table 1006 is "0", it will be judged that blood glucose measurement and insulin administration are both forgotten to be performed on the patient.
When the value of the "ROUND TYPE IN PROCESS" field of the patient information table 1004 is "3" which means "performing blood glucose measurement and insulin administration", in the case where the value of the "INSULIN ADMINISTRATION RESULT" field of the patient history table 1006 is "1", it will be judged that insulin administration is forgotten to be performed on the patient.

By comparing the value of the "ROUND TYPE IN PROCESS" field of the patient information table 1004 with the value of the "INSULIN ADMINISTRATION RESULT" field of the patient history table 1006 in the aforesaid manner, the measurement data managing device 104 judges whether or not the content due to be performed on the patient has been performed.
By automatically recreating the round data and transmitting the recreated round data to the blood glucose meter 102 for the patient whose blood glucose measurement or insulin administration is forgotten to be performed, failing to perform blood glucose measurement or insulin administration can be prevented. At this time, since the operation of creating the round data is automatically performed by the measurement data managing device 104, reround can be quickly performed without performing inconvenient operation such as for the user to reconfirm the patient whose blood glucose measurement or insulin administration is forgotten to be performed.

The round data can be recreated as follows.
(1) In the case of performing only blood glucose measurement or performing only insulin administration, the same data will be transmitted as it is.
(2) In the case where, although both the blood glucose measurement and the insulin administration are instructed, the both instructed operations have not been performed, the same data will be transmitted as it is.
(3) In the case where, although both the blood glucose measurement and the insulin administration are instructed, only the blood glucose measurement has been performed, and the insulin administration has not been performed, the data of insulin administration only will be created and transmitted.
   At this time, as the latest data, the blood glucose level of the patient included in the patient's measurement data 117 received from the blood glucose meter 102 is recorded in the "PATIENT MEASUREMENT HISTORY DATA" field of the newly created patient data 114.
   Upon detecting the fact that the "INSULIN THERAPY CLASSIFICATION" field of the patient data 114 is set to "5", which means only performing "insulin administration using sliding scale", the blood glucose meter 102 reads out the latest blood glucose level from the "PATIENT MEASUREMENT HISTORY DATA" field. Further, the sliding scale 2509 in the SLIDING SCALE ADMINISTRATION INFORMATION is referred (searched) using the reads out blood glucose level to acquire dosage and kind of the insulin due to be administered to the patient.

The blood glucose measuring system is disclosed in the present embodiment.
The measurement data managing device holds the type information of the content to be performed, which is contained in the round data transmitted from the measurement data managing device to the blood glucose meter, in the patient information table thereof. Further, after writing the data received from the blood glucose meter in the patient history table, the measurement data managing device verifies whether or not blood glucose measurement and/or insulin administration has been performed according to the instruction. As a result of the verification, in there is any discrepancy, it can be judged that blood glucose measurement and/or insulin administration is forgotten to be performed. Based on this judgment, the measurement data managing device displays a warning message on the display unit to tell the user such as a nurse that the there is a patient whose blood glucose measurement and/or insulin administration is forgotten to be performed.
In response to the warning message, the user automatically creates the patient whose blood glucose measurement and/or insulin administration is forgotten to be performed and the content due to be performed on the patient again, and transmits the round data to the blood glucose meter.
With such the aforesaid configuration, it is possible to quickly grasp whether or not there is any patient whose blood glucose measurement and/or insulin administration is forgotten to be performed. Further, reround can be quickly performed.

Although the present embodiment of the present invention is described above, it should be noted that the present invention is not limited to the above embodiment but includes various other modifications and applications without departing from the spirit of the claims of the present invention.

### Explanation of Reference Numerals

- 101: blood glucose measuring system
- 102: blood glucose meter
- 103: cradle
- 104: measurement data managing device
- 105: USB cable
- 112: internal patient table
- 114: patient data
- 115: user's basic data
- 116: tip lot data
- 117: patient's measurement data
- 202: optical measuring section
- 203: LCD
- 204: power switch
- 205: Cursor keys
- 206: Enter key
- 207: bar-code key
- 208: bar-code reader
- 209: power terminal
- 210: infrared communication window
- 211: battery lid
- 212: measuring tip
- 302: eject lever
- 402: charging terminal
- 403: infrared communication window
- 602: CPU
- 603: ROM
- 604: RAM
- 605: bus
- 606: thermistor
- 607: calendar clock
- 608: operating section
- 609: light-emitting diode
- 610: driver
- 611: D/A converter
- 612: phototransistor
- 613: A/D converter
- 614: nonvolatile storage
- 615: display unit
- 616: buzzer
- 617: infrared communication section
- 618: power circuit
- 702: CPU
- 703: ROM
- 704: RAM
- 705: bus
- 706: USB interface
- 717: infrared communication section
- 718: charging circuit
- 902: bus
- 903: CPU
- 904: ROM
- 905: RAM
- 906: nonvolatile storage
- 907: display unit
- 908: USB interface
- 909: operating section
- 1002: display control section
- 1003: data edit processing section
- 1004: patient information table
- 1005: insulin administration instruction table
- 1006: patient history table
- 1007: scheduler
- 1008: calendar clock
- 1009: incomplete patient history processing section
- 1010: incomplete patient warning processing section
- 1012: blood glucose meter detection section
- 1011: complete patient history processing section
- 1202: top menu window
- 1203: blood glucose meter state display column
- 1204: "TRANSFER ROUND DATA" button
- 1205: "TRANSFER METER SETTING" button
- 1302: blood glucose measurement table receiving section
- 1303: complete patient temporary file
- 1304: incomplete patient temporary file
- 1305: control section
- 1702: warning window
- 1703: "UNSUBJECTED PATIENT LIST" display column
- 1704: "REMEASURE" button
- 1705: "TRANSFER" button
- 1802: search section
- 1803: temporary file
- 1804: additional recording section
- 2002: search section
- 2003: temporary file
- 2302: warning message
- 2402: incomplete patient list window
- 2403: "CONFIRM" button
- 2404: "RETURN" button
- 2502: patient
- 2503: patient ID
- 2504: nurse
- 2505: user ID
- 2506: box
- 2507: tip lot number
- 2508: syringe
- 2509: sliding scale
- 2902: input/output control section
- 2903: display control section
- 2904: patient data converter
- 2905: patient measurement data converter
- 3302: blood glucose meter detecting section
- 3303: blood glucose meter operating section
- 3304: user interface control section
- 3305: table input/output section
- 3306: patient measurement temporary file
- 3702: main menu window
- 3703: blood glucose meter state display column
- 3704: illustration
- 3705: connection state display column
- 3706: nickname display column
- 3708: warning message

## Claims

1. A blood glucose measuring system comprising:
a blood glucose meter; and
a measurement data managing device,
wherein the blood glucose meter comprises:
a measuring portion for measuring blood glucose level;
a nonvolatile storage adapted to store a blood glucose measurement table for recording the measured blood glucose level; and
a transmitting section adapted to transmit the blood glucose measurement table, and
wherein the measurement data managing device comprises:
a insulin administration instruction table having a patient ID field, and a scheduled measurement time field for performing blood glucose measurement;
a patient history table having a patient ID field, a scheduled measurement date and time field, a measurement date and time field, a blood glucose level field, and a incomplete flag field;
a receiving section for being connected with the blood glucose meter to receive the blood glucose measurement table from the blood glucose meter;
a complete patient history processing section adapted to record a record acquired from the blood glucose measurement table in the patient history table as a record whose incomplete flag field is "false";
an incomplete patient history processing section adapted to additionally record a record, which is obtained by searching the scheduled measurement time field of the insulin administration instruction table for the patient ID scheduled to receive a blood glucose measurement in a period between the current time and a predetermined time ago, in the patient history table as a record whose incomplete flag field is "true";
an incomplete patient warning processing section adapted to search the patient history table to judge whether or not there is any record whose incomplete flag field is "true";
a display control section adapted to create a warning in the case where the incomplete patient warning processing section judges that there is a record whose incomplete flag field is "true" in the patient history table; and
a display unit adapted to display the warning created by the display control section.

2. The blood glucose measuring system according to claim 1, wherein the complete patient history processing section is adapted to set the incomplete flag field of the record additionally recorded in the patient history table by the incomplete patient history processing section to "false".

3. The blood glucose measuring system according to claim 1, wherein the complete patient history processing section is adapted to detect the blood-glucose-unmeasured patient in the received blood glucose measurement table, and make the display control section to display a warning.

4. A blood glucose measuring system comprising:
a blood glucose meter; and
a measurement data managing device,
wherein the blood glucose meter comprises:
a measuring portion for measuring blood glucose level;
a nonvolatile storage adapted to store a blood glucose measurement table for recording the measured blood glucose level; and
a transmitting section adapted to transmit the blood glucose measurement table, and
wherein the measurement data managing device comprises:
a insulin administration instruction table having a patient ID field, and a scheduled measurement time field for performing blood glucose measurement;
a patient history table having a patient ID field, a scheduled measurement date and time field, a measurement date and time field, and a blood glucose level field;
a receiving section for being connected with the blood glucose meter to receive the blood glucose measurement table from the blood glucose meter;
a complete patient history processing section adapted to record a record acquired from the blood glucose measurement table in the patient history table as a record whose incomplete flag field is "false", and detect if there is any blood-glucose-unmeasured patient in the received blood glucose measurement table;
a display control section adapted to create a warning in the case where the complete patient history processing section has detected the existence of the blood-glucose-unmeasured patient in the received blood glucose measurement table; and
a display unit adapted to display the warning created by the display control section.

5. A measurement data managing device comprising:
a patient information table having a round type in process field in which an instruction on whether or not blood glucose measurement shall be performed and whether or not drug administration shall be performed is recorded for each patient, the instruction being contained in patient data transmitted to a blood glucose meter;
a patient history table having a drug administration result field in which results of performing the blood glucose measurement and the drug administration are recorded for each patient, the results being contained in patient's measurement data received from the blood glucose meter; and
a control section adapted to compare the round type in process field with the drug administration result field, and, if there is any discrepancy, create necessary data for performing the blood glucose measurement and/or the drug administration for each patient associated with the discrepancy, as well as create a warning message, and transmit the created data to the blood glucose meter.

6. The measurement data managing device according to claim 5, wherein the control section is adapted to transmit data to the blood glucose meter to instruct it to only perform drug administration, and record the data in the patient information table in the case where the value of the round type in process field represents an instruction of performing both the blood glucose measurement and the drug administration, but the value of the drug administration result field shows that only the blood glucose measurement has been performed.

7. A blood glucose measuring system comprising:
a blood glucose meter; and
a measurement data managing device,
wherein the blood glucose meter comprises:
a patient measurement data converter adapted to record patient data received from the outside in a storage, the patient data having an instruction on whether or not blood glucose measurement shall be performed and whether or not drug administration shall be performed recorded therein for each patient;
a input/output control section adapted to record information on results of performing the blood glucose measurement and the drug administration based on the patient data in the storage for each patient; and
a patient data converter adapted to transmit the information on the results to the outside as patient's measurement data, and
wherein the measurement data managing device comprises:
a patient information table having a round type in process field in which the instruction on whether or not blood glucose measurement shall be performed and whether or not drug administration shall be performed is recorded for each patient, the instruction being contained in the patient data transmitted to the blood glucose meter;
a patient history table having a drug administration result field in which the results of performing the blood glucose measurement and the drug administration are recorded for each patient, the results being contained in the patient's measurement data received from the blood glucose meter; and
a control section adapted to compare the round type in process field with the drug administration result field, and, if there is any discrepancy, create necessary patient data for each patient associated with the discrepancy, as well as create a warning message, and transmit the created patient data to the blood glucose meter.
